# EUROPEAN PATENT APPLICATION

(11) **EP 4 299 592 A1**
(43) Date of publication of application: **03.01.2024**
(21) Application number: 22758884.5
(22) Date of filing: 23.02.2022
(51) Int. Cl.: C07K 16/28, C12N 15/13, C12N 5/10, A61P 35/00

(54) **CO-EXPRESSED CXCR2 AND T CELLS OF STAR SPECIFIC TO GPC3, AND USE THEREOF**

(30) Priority: 25.02.2021 CN 202110214637
(71) Applicant: Bristar Immunotech Limited, Beijing 102206 (CN); Tsinghua University, Beijing 100084 (CN)
(72) Inventor: RUI, Wei, Beijing 102206 (CN); LIU, Guangna, Beijing 102206 (CN); ZHENG, Hongli, Beijing 102206 (CN); WANG, Jiasheng, Beijing 100084 (CN); TONG, Dan, Beijing 102206 (CN); ZHAO, Xueqiang, Beijing 102206 (CN); LIN, Xin, Beijing 100084 (CN)
(74) Representative: Michalski Hüttermann & Partner Patentanwälte mbB
(86) International application number: PCT/CN2022/077434
(87) International publication number: WO 2022/179520

(57) **Abstract**

The invention relates to the field of biomedicine, in particular to a T cell co-expressing CXCR2 and a Synthetic T-Cell Receptor and Antigen Receptor (STAR) against GPC3, and uses thereof.

## Description

### Technical field

The invention relates to the field of biomedicine, in particular to a T cell co-expressing CXCR2 and a Synthetic T-Cell Receptor and Antigen Receptor (STAR) against GPC3, and uses thereof.

### Background

Cell therapy, especially T cell-related therapy, has developed rapidly, among which chimeric antigen receptor T cell (CAR-T) therapy and TCR-T therapy have attracted much attention.

TCR-T therapy is based on the T cell receptor (TCR). TCR is the identity of T cells, which can be divided into αβ T cells and γδ T cells based on the type of TCR. During the development, a T precursor cell will undergo VDJ rearrangement of TCR γ and TCR δ chains, which, if rearrangement is successful, will develop into a γδ T cell, or if rearrangement ends in failure, will undergo VDJ recombination of TCR α and TCR β chains, and then develop into a αβ T cell . αβ T cells account for 90%-95% of peripheral blood T cells, while γδ T cells account for 5%-10% of peripheral blood T cells. The two types of T cells recognize antigens in MHC-restricted and MHC-unrestricted ways, respectively, which play an important role in the immunity to pathogens and tumors.

The T cell receptor (TCR) complex molecule contains multiple chains, in which TCR α and TCR β chains (or TCR γ and TCR δ chains) are responsible for recognizing MHC-polypeptide molecules, and the other six CD3 subunits bind to TCR α/β chains (or TCR γ/δ chains) to play the role of signal transduction. The natural TCR complex contains ten ITAM signal sequences, which can transmit stronger signals than CAR in theory. By employing the signal transduction function of natural TCR, it is possible to construct a new receptor to alleviate T cell disability, which can play a better role against solid tumor. The ectodomain of TCR is very similar to the Fab domain of an antibody, so the variable region sequence of TCR can be replaced by a variable region sequence of an antibody, so as to obtain a Synthetic TCR and Antigen Receptor (STAR), which not only has antibody specificity, but also has superior signal transduction function of a natural TCR on mediating T cell activation.

The number of cell therapies in the field that have been put into clinical application is quite limited, so there is still a need for improved T cell therapies, such as STAR-based T cell therapies.

### Summary of the invention

In one aspect, the invention provides an isolated therapeutic T cell co-expressing CXCR2 and a Synthetic T cell Receptor and Antigen Receptor (STAR) against GPC3, wherein the STAR against GPC3 comprises an antigen binding region against GPC3, and the STAR against GPC3 comprises an α chain comprising a first constant region and a β chain comprising a second constant region.

In some embodiments,
i) the α chain comprises an antigen binding region against GPC3 and a first constant region, the β chain comprises a second constant region; or
ii) the α chain comprises a first constant region, the β chain comprises an antigen binding region against GPC3 and a second constant region;
iii) the α chain comprises a first antigen binding region against GPC3 and a first constant region, the β chain comprises a second antigen binding region against GPC3 and a second constant region.

In some embodiments, the first constant region is a native TCR α chain constant region, e.g., a native human TCR α chain constant region or a native mouse TCR α chain constant region. In some embodiments, the first constant region is a modified TCR α chain constant region. In some embodiments, the modified TCRα chain constant region is derived from the mouse TCRα chain constant region, and the amino acid at position 48, such as threonine T, is mutated to cysteine C, as compared to the wild-type mouse TCRα chain constant region. In some embodiments, the modified TCR α chain constant region is derived from the mouse TCR α chain constant region, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCRα chain constant region. In some embodiments, the modified TCR α chain constant region is derived from the mouse TCR α chain constant region, the amino acid at position 6 such as E is substituted by D, and the amino acid K at position 13 is substituted by R, and amino acids at positions 15-18 are deleted, as compared to the wild-type mouse TCRα chain constant region. In some embodiments, the modified TCRα chain constant region is derived from the mouse TCRα chain constant region, the amino acid at position 48, such as threonine T, is mutated to Cysteine C, the amino acid at position 112 such as serine S is mutated to leucine L, the amino acid at position 114 such as methionine M is mutated to isoleucine I, the amino acid at position 115 such as Glycine G is mutated to valine V, as compared to the wild-type mouse TCRα chain constant region. In some embodiments, the modified TCRα chain constant region is derived from the mouse TCRα chain constant region, the amino acid at position 6, such as E, is substituted by D, and the amino acid K at position 13 is substituted by R, the amino acids at positions 15-18 are deleted, the amino acid at position 48 such as threonine T is mutated to cysteine C, and the amino acid at position 112 such as serine S is mutated to leucine acid L, the amino acid at position 114 such as methionine M is mutated to isoleucine I, and the amino acid at position 115 such as glycine G is mutated to valine V, as compared to the wild-type mouse TCRα chain constant region. In some embodiments, the modified TCR α chain constant region is derived from the mouse TCR α chain constant region, and lacks the endodomain of the constant region, for example, the amino acids at positions 136-137 are deleted, as compared to the wild-type mouse TCRα chain constant region. In some embodiments, the modified TCRα chain constant region comprises an amino acid sequence shown in one of SEQ ID NOs: 3-4 and 28-30.

In some embodiments, the second constant region is a native TCR β chain constant region, e.g., a native human TCR β chain constant region or a native mouse TCR β chain constant region. In some embodiments, the second constant region is a modified TCR β chain constant region. In some embodiments, the modified TCR β chain constant region is derived from the mouse TCR β chain constant region, and the amino acid at position 56, such as serine S, is mutated to cysteine C, as compared to the wild-type mouse TCRβ chain constant region. In some embodiments, the modified TCR β chain constant region is derived from the mouse TCR β chain constant region, the amino acid at position 3 such as R is substituted by K, the amino acid at position 6 such as T is substituted by F, the amino acid K at position 9 is substituted by E, S at position 11 is substituted by A, L at position 12 is substituted by V, and amino acids at positions 17 and 21-25 are deleted, as compared to the wild-type mouse TCRβ chain constant region. In some embodiments, the modified TCR β chain constant region is derived from a mouse TCR β chain constant region, the amino acid at position 56 such as serine S is mutated to cysteine C, the amino acid at position 3 such as R is substituted by K, the amino acid at position 6 such as T is substituted by F, K at position 9 is substituted by E, S at position 11 is substituted by A, and L at position 12 is substituted by V, and amino acids 17, 21-25 are deleted, as compared to the wild-type mouse TCRβ chain constant region. In some embodiments, the modified TCR β chain constant region is derived from the mouse TCR beta chain constant region, and lacks the endodomain of the constant region, for example, the amino acids at positions 167-172 are deleted, as compared to the wild-type mouse TCRβ chain constant region. In some embodiments, the modified TCRβ chain constant region comprises an amino acid sequence shown in one of SEQ ID NOs:7-8 and 31-33.

In some embodiments, the antigen-binding region against GPC3 comprises a heavy chain variable region of an antibody specifically binding to the target antigen GPC3 and a light chain variable region of said antibody. In some embodiments, the first antigen binding region comprises a heavy chain variable region of an antibody that specifically binds to target antigen GPC3, and the second antigen binding region comprises a light chain variable region of said antibody; or the first antigen binding region comprises a light chain variable region of an antibody that specifically binds to target antigen GPC3, and the second antigen binding region comprises a heavy chain variable region of said antibody. In some embodiments, the heavy chain variable region comprises VH CDR1 shown in SEQ ID NO:22, VH CDR2 shown in SEQ ID NO:23, and VH CDR3 shown in SEQ ID NO:24, the light chain variable region comprises VL CDR1 shown in SEQ ID NO:25, VL CDR2 shown in SEQ ID NO:26, and VL CDR3 shown in SEQ ID NO:27. In some embodiments, the heavy chain variable region comprises the amino acid sequence shown in SEQ ID NO:9, and the light chain variable region comprises the amino acid sequence shown in SEQ ID NO:10.

In some embodiments, the α chain and/or β chain has at least one exogenous intracellular functional domain linked to its C-terminus. In some embodiments, the exogenous intracellular functional domain is linked directly or via a linker to the C-terminus of the constant region of the α chain and/or β chain. In some embodiments, the exogenous intracellular functional domain is linked to the C-terminus of the constant region of the α-chain and/or β-chain whose endodomain is deleted, through a linker. In some embodiments, the linker is a (G4S)n linker, where n represents an integer from 1-10, preferably, n is 3.

In some embodiments, the exogenous intracellular functional domain is an endodomain of a co-stimulatory molecule, preferably an endodomain of OX40, for example, the endodomain of OX40 comprises the amino acid sequence of SEQ ID NO: 11.

In some embodiments, the α chain comprises an amino acid sequence shown in SEQ ID NO: 13, 15, 17 or 19. In some embodiments, the β chain comprises an amino acid sequence shown in SEQ ID NO: 14, 16, 18 or 20. In some embodiments, the STAR comprises an α chain set forth in SEQ ID NO:13 and a β chain set forth in SEQ ID NO:14; or the STAR comprises an α chain set forth in SEQ ID NO:15 and a β chain set forth in SEQ ID NO:16; or the STAR comprises an α chain set forth in SEQ ID NO: 17 and a β chain set forth in SEQ ID NO:18; or the STAR comprises an α chain set forth in SEQ ID NO:19 and a β chain set forth in SEQ ID NO:20; or the STAR comprises an α chain set forth in SEQ ID NO:15 and a β chain set forth in SEQ ID NO:14; or the STAR comprises an α chain set forth in SEQ ID NO:19 and a β chain set forth in SEQ ID NO:18.

In one aspect, the invention provides a pharmaceutical composition, which comprises the therapeutic T cell of the present invention and a pharmaceutically acceptable carrier.

In one aspect, the invention provides the use of the therapeutic T cell of the present invention or the pharmaceutical composition of the present invention in the preparation of a medicine for the treatment of a disease such as cancer in a subject. In some embodiments, the disease is a GPC3-related disease, such as liver cancer such as hepatocellular carcinoma, lung cancer such as lung squamous cell carcinoma (SqCC), gastric cancer, ovarian cancer, melanoma or pediatric embryonal tumor.

### Brief description of the drawings

Figure 1. shows the maps of some constructs used in the examples.
Figure 2. shows the killing effect of T cells co-expressing CAR against GPC3 antigen or STARs against GPC3 antigen with different constant region optimized structures (A and C: Nrec-Cys-TM; B and D: Cys-TM) and CXCR2 on target cells, and the secretion of cytokines under the stimulation of target cells.
Figure 3. shows that expression of CXCR2 increases T cell infiltration into tumors.
Figure 4. shows the in vivo tumor suppressive effect of T cells co-expressing CXCR2 and CAR targeting GPC3 antigen or STAR targeting GPC3 antigen with optimized constant region structure of Nrec-Cys-TM.
Figure 5. In vitro functional evaluation of GPC3 STAR-aOX40-CXCR2-T with optimized constant region structure of Cys-TM compared to corresponding GPC3 STAR-aOX40-T. A: Construction strategy of GPC3 STAR-aOX40-CXCR2-T containing TRAC (Cys-TM) and TRBC (Cys-TM); B: GPC3 STAR-aOX40-CXCR2-T showed good co-expression level of GPC3 and CXCR2; C: The tumor cell killing activity of GPC3 STAR-aOX40-CXCR2-T compared with GPC3 STAR-aOX40-T and GPC3 CAR-T.
Figure 6. shows the in vitro migration ability of GPC3 STAR-aOX40-CXCR2-T with optimized constant region structure of Cys-TM.
Figure 7. shows the ability of GPC3 STAR-aOX40-CXCR2-T with the optimized constant region structure of Cys-TM to migrate to tumors in vivo. A: Experimental process; B: Comparison of tumor tissue weight after cell reinfusion; C: Comparison of infiltrating T cells in tumor tissues.
Figure 8. shows that the anti-tumor activity of GPC3 STAR-aOX40-CXCR2-T with optimized constant region structure of Cys-TM is stronger than that of corresponding GPC3 STAR-aOX40-T.

### Detailed description of the invention

Unless otherwise indicated or defined, all used terms have the common meaning in the field, which will be understood by those skilled in the field. See, for example, the standard manual, such as Sambrook et al., "Molecular cloning: a laboratory manual"; Lewin, "Genes VIII", and Roitt et al., "Immunology" (8nd edition), and the general prior art cited herein; in addition, unless otherwise stated, all the methods, steps, techniques and operations that are not specifically detailed may and have been performed in a manner known per se, which will be understood by those skilled in the art. Also see, for example, the standard manual, the above general prior art and other references cited therein.

As used herein, the term "and/or" encompasses all combinations of items connected by the term, which shall be deemed to have been separately listed herein. For example, "A and/or B" covers "A", "A and B", and "B". For example, "A, B and/or C" covers "A", "B", "C", "A and B", "A and C", "B and C", and "A and B and C".

The term "comprising" is used herein to describe a sequence of a protein or nucleic acid, which may consist of said sequence, or may have additional amino acids or nucleotides at one or both ends of said protein or nucleic acid but still possess the activity described herein. In addition, those skilled in the art will understand that methionine encoded by the start codon at the N-terminal of a polypeptide is retained in certain practical situations (e.g., when expressed in a particular expression system), but does not substantially affect the function of the polypeptide. Thus, in the description of a specific polypeptide amino acid sequence, while it may not contain methionine encoded by the start codon at its N-terminal, but still covers a sequence comprising the methionine by the time, and correspondingly, the coding nucleotide sequences thereof may also contain the start codon; and vice versa.

As used herein, "the amino acid number being made reference to SEQ ID NO: x" (SEQ ID NO: x is a specific sequence listed herein) means that the position number of a particular amino acid described is the position number of the amino acid corresponding to that amino acid on SEQ ID NO: x. The amino acid correspondence between different amino acid sequences can be determined by sequence alignment methods known in the art. For example, the amino acid correspondence can be determined by an EMBL-EBI online alignment tool (https://www.ebi.ac.uk/Tools/psa/), in which two sequences can be aligned using Needleman-Wunsch algorithm with default parameters. For example, if amino acid at position 46 starting from the N-terminal of a polypeptide is aligned in sequence alignment with an amino acid at position 48 of SEQ ID NO: x, then the amino acid in the polypeptide may also be described herein as "alanine at position 48 of the polypeptide, the amino acid position being made reference to SEQ ID NO: x". In the present invention, reference is made to SEQ ID NO: 2 for the amino acid position related to α chain constant region. In the present invention, reference is made to SEQ ID NO: 6 for the amino acid position related to β chain constant region.

CXCR2 belongs to the G protein-coupled receptor family, and is often distributed in neutrophils, and can also be found in mast cells, macrophages, endothelial cells and various tumor cells. Because of its high affinity with interleukin (IL)-8 Specific binding, it is also called IL8Rβ. The CXCR2 coding gene is located on chromosome 2q33-q36, and the receptor protein consists of about 350 amino acids. The ligands of CXCR2 are various CXC chemokines such as CXCL1, CXCL2, CXCL3, CXCL5, CXCL6, CXCL7, and CXCL8. Early studies have found that CXCR2 can recruit neutrophils to transfer to inflammatory areas, which is considered to be related to inflammatory immunity. In recent years, it has been found that CXCR2 is involved in the occurrence, development and treatment of various tumors. An exemplary amino acid sequence of CXCR2 is shown in SEQ ID NO:12. CXCR2 described herein also encompasses functional fragments or variants thereof. For example, the variant comprises an amino acid sequence having at least 75%, at least 80%, at least 90%, at least 95%, at least 96%, at least 97%, at least 98%, at least 99%, 99.5% identity to SEQ ID NO: 12.

GPC3 (Glypican 3) is involved in cell division, or regulation of cell division patterns during development. GPC3 is highly expressed in HCC (hepatocellular carcinoma) cells and thus has been used as a diagnostic marker and therapeutic target for HCC.

The present inventors surprisingly found that co-expressing CXCR2 and a synthetic T cell receptor and antigen receptor (STAR) targeting GPC3 (Glypican 3), especially a STAR engineered in the constant region, in T cells, the target killing ability of T cells can be significantly enhanced and their therapeutic efficacy can be improved.

In one aspect, the invention provides an isolated therapeutic T cell co-expressing CXCR2 and a Synthetic T cell Receptor and Antigen Receptor (STAR) against GPC3, wherein the STAR against GPC3 comprises an antigen binding region against GPC3, and the STAR against GPC3 comprises an αchain comprising a first constant region and a βchain comprising a second constant region.

In some embodiments, the T cell co-expresses CXCR2 and a Synthetic T cell Receptor and Antigen Receptor (STAR) against GPC3, wherein the STAR against GPC3 comprises an α chain and a β chain, the α chain comprises an antigen binding region against GPC3 and a first constant region, the β chain comprises a second constant region.

In some embodiments, the T cell co-expresses CXCR2 and a Synthetic T cell Receptor and Antigen Receptor (STAR) against GPC3, wherein the STAR against GPC3 comprises an α chain and a β chain, the α chain comprises a first constant region, the β chain comprises an antigen binding region against GPC3 and a second constant region.

In some embodiments, the T cell co-expresses CXCR2 and a Synthetic T cell Receptor and Antigen Receptor (STAR) against GPC3, wherein the STAR against GPC3 comprises an α chain and a β chain, the α chain comprises a first antigen binding region against GPC3 and a first constant region, the β chain comprises a second antigen binding region against GPC3 and a second constant region.

In some embodiments, the co-expression is co-overexpression. For example, the expression of CXCR2 in the isolated therapeutic T cell of the invention is increased, preferably significantly increased, relative to an unmodified control T cell (e.g., a naturally occurring T cell).

In some embodiments, the antigen binding region is fused directly or indirectly (e.g., via a linker) to the N-terminus of the constant region.

In some embodiments, the α and β chains are capable of forming a functional TCR complex upon expression in a T cell. For example, upon expression in a T cell, the α chain and β chain can combine with endogenous CD3 molecules (CD3εδ, CD3γε, CD3ζζ) to form a TCR complex of 8 subunits, and the TCR complex is displayed on the cell surface, and activates the T cell upon binding to the target antigen GPC3. A functional TCR complex refers to that it can activate the T cell after specifically binding to a target antigen.

In some embodiments, the first constant region is a native TCR α chain constant region, e.g., a native human TCR α chain constant region or a native mouse TCR α chain constant region. An exemplary native human TCRα chain constant region comprises the amino acid sequence shown in SEQ ID NO:1. An exemplary native mouse TCRα chain constant region comprises the amino acid sequence shown in SEQ ID NO:2.

In some embodiments, the first constant region is a modified TCR α chain constant region.

In some embodiments, the modified TCRα chain constant region is derived from the mouse TCRα chain constant region, and the amino acid at position 48, such as threonine T, is mutated to cysteine C, as compared to the wild-type mouse TCRα chain constant region.

In some embodiments, the modified TCR α chain constant region is derived from the mouse TCR α chain constant region, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCRα chain constant region.

In some embodiments, the modified TCR α chain constant region is derived from the mouse TCR α chain constant region, the amino acid at position 6 such as E is substituted by D, and the amino acid K at position 13 is substituted by R, and amino acids at positions 15-18 are deleted, as compared to the wild-type mouse TCRα chain constant region.

In some embodiments, the modified TCRα chain constant region is derived from the mouse TCRα chain constant region, the amino acid at position 48, such as threonine T, is mutated to Cysteine C, the amino acid at position 112 such as serine S is mutated to leucine L, the amino acid at position 114 such as methionine M is mutated to isoleucine I, the amino acid at position 115 such as Glycine G is mutated to valine V, as compared to the wild-type mouse TCRα chain constant region.

In some embodiments, the modified TCRα chain constant region is derived from the mouse TCRα chain constant region, the amino acid at position 6, such as E, is substituted by D, and the amino acid K at position 13 is substituted by R, the amino acids at positions 15-18 are deleted, the amino acid at position 48 such as threonine T is mutated to cysteine C, and the amino acid at position 112 such as serine S is mutated to leucine acid L, the amino acid at position 114 such as methionine M is mutated to isoleucine I, and the amino acid at position 115 such as glycine G is mutated to valine V, as compared to the wild-type mouse TCRα chain constant region.

In some embodiments, the modified TCR α chain constant region lacks the endodomain of the constant region, for example, the amino acids at positions 136-137 are deleted, as compared to the wild-type TCRα chain constant region.

In some embodiments, the modified TCR α chain constant region is derived from the mouse TCR α chain constant region, and lacks the endodomain of the constant region, for example, the amino acids at positions 136-137 are deleted, as compared to the wild-type mouse TCRα chain constant region.

In some specific embodiments, the modified TCRα chain constant region comprises an amino acid sequence shown in one of SEQ ID NOs: 3-4 and 28-30.

In some embodiments, the second constant region is a native TCR β chain constant region, e.g., a native human TCR β chain constant region or a native mouse TCR β chain constant region. An exemplary native human TCR β chain constant region comprises the amino acid sequence shown in SEQ ID NO:5. An exemplary native mouse TCR β chain constant region comprises the amino acid sequence shown in SEQ ID NO:6.

In some embodiments, the second constant region is a modified TCR β chain constant region.

In some embodiments, the modified TCR β chain constant region is derived from the mouse TCR β chain constant region, and the amino acid at position 56, such as serine S, is mutated to cysteine C, as compared to the wild-type mouse TCRβ chain constant region.

In some embodiments, the modified TCR β chain constant region is derived from the mouse TCR β chain constant region, the amino acid at position 3 such as R is substituted by K, the amino acid at position 6 such as T is substituted by F, the amino acid K at position 9 is substituted by E, S at position 11 is substituted by A, L at position 12 is substituted by V, and amino acids at positions 17 and 21-25 are deleted, as compared to the wild-type mouse TCRβ chain constant region.

In some embodiments, the modified TCR β chain constant region is derived from a mouse TCR β chain constant region, the amino acid at position 56 such as serine S is mutated to cysteine C, the amino acid at position 3 such as R is substituted by K, the amino acid at position 6 such as T is substituted by F, K at position 9 is substituted by E, S at position 11 is substituted by A, and L at position 12 is substituted by V, and amino acids 17, 21-25 are deleted, as compared to the wild-type mouse TCRβ chain constant region.

In some embodiments, the modified TCR β chain constant region lacks the endodomain of the constant region, for example, the amino acids at positions 167-172 are deleted, as compared to the wild-type TCRβ chain constant region.

In some embodiments, the modified TCR β chain constant region is derived from the mouse TCR beta chain constant region, and lacks the endodomain of the constant region, for example, the amino acids at positions 167-172 are deleted, as compared to the wild-type mouse TCRβ chain constant region.

In some specific embodiments, the modified TCRβ chain constant region comprises an amino acid sequence shown in one of SEQ ID NOs:7-8 and 31-33.

In some embodiments, the first constant region comprises the amino acid sequence shown in SEQ ID NO:28, and the second constant region comprises the amino acid sequence shown in SEQ ID NO:7. In some embodiments, the first constant region comprises the amino acid sequence shown in SEQ ID NO:29, and the second constant region comprises the amino acid sequence shown in SEQ ID NO:7. In some embodiments, the first constant region comprises the amino acid sequence shown in SEQ ID NO:30, and the second constant region comprises the amino acid sequence shown in SEQ ID NO:8.

As used herein, "antigen-binding region" means that it alone or in combination with another antigen-binding region can specifically bind to a target antigen. In some embodiments, the antigen binding region is derived from an antibody that specifically binds to the target antigen, including any commercially available antibody.

In some embodiments, the first antigen binding region comprises a heavy chain variable region of an antibody that specifically binds to target antigen GPC3, and the second antigen binding region comprises a light chain variable region of said antibody. In some embodiments, the first antigen binding region comprises a light chain variable region of an antibody that specifically binds to target antigen GPC3, and the second antigen binding region comprises a heavy chain variable region of said antibody.

In some embodiments, the heavy chain variable region comprises VH CDR1 shown in SEQ ID NO:22, VH CDR2 shown in SEQ ID NO:23, and VH CDR3 shown in SEQ ID NO:24, the light chain variable region comprises VL CDR1 shown in SEQ ID NO:25, VL CDR2 shown in SEQ ID NO:26, and VL CDR3 shown in SEQ ID NO:27.

In some embodiments, the heavy chain variable region comprises the amino acid sequence shown in SEQ ID NO:9, and the light chain variable region comprises the amino acid sequence shown in SEQ ID NO:10.

In some embodiments, the antigen-binding region may be a single-chain antibody (such as scFv) or a single-domain antibody (such as a camelid antibody) that specifically binds to the target antigen GPC3. In this case, for example, said first and second antigen binding regions may independently bind to a same or different epitopes of GPC3. In some embodiments, the α chain of the STAR comprises an antigen binding region specific against GPC3 and the β chain does not comprise an antigen binding region specific against GPC3. In some embodiments, the α chain of the STAR does not comprise an antigen binding region specific against GPC3 and the β chain comprises an antigen binding region specific against GPC3. In some embodiments, the α chain and/or β chain, preferably the α chain has at least one exogenous intracellular functional domain linked to its C-terminus. In some embodiments, the exogenous intracellular functional domain is linked directly or via a linker to the C-terminus of the constant region of the α chain and/or β chain, preferably the α chain. In some embodiments, the exogenous intracellular functional domain is linked to the C-terminus of the constant region of the α-chain and/or β-chain preferably the α-chain whose endodomain is deleted, through a linker. In some embodiments, the linker is a (G4S)n linker, where n represents an integer from 1-10, preferably, n is 3.

In some embodiments, the first constant region is a modified TCRα chain constant region, which is derived from a mouse TCRα chain constant region, and the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region, and the modified TCR α chain constant region lacks the endodomain of the constant region, for example, amino acids at positions 136-137 are deleted, as compared to the wild-type mouse TCRα chain constant region, and the α chain comprises the endodomain of OX40 linked to the C-terminus of the constant region (for example, linked by a linker, such as (G4S)n linker, wherein n represents an integer from 1 to 10, preferably, n is 3); and

the second constant region is a modified TCR βchain constant region, which is derived from a mouse TCR β chain constant region, and the amino acid at position 56, such as serine S, is mutated to Cystine C, as compared to the wild-type mouse TCR β chain constant region,.

As used herein, "exogenous" means a protein or nucleic acid sequence from a foreign species, or, if from the same species, a protein or nucleic acid sequence whose composition and/or location has been significantly altered from its native form by deliberate human intervention.

As used herein, an "exogenous intracellular domain" may be an intracellular domain of a co-stimulatory molecule such as CD40, OX40, ICOS, CD28, 4-1BB, CD27, CD137; it may also be a intracellular domain of a co-suppressive molecule, such as those of TIM3, PD1, CTLA4, LAG3; it may also be intracellular domain of cytokine receptors such as interleukin receptors (such as IL-2β receptor, IL-7α receptor or IL- 21 receptors), interferon receptors, tumor necrosis factor superfamily receptors, colony-stimulating factor receptors, chemokine receptors, growth factor receptors, or other membrane proteins; or domain of intracellular proteins such as NIK.

In some preferred embodiments, the exogenous intracellular functional domain is an endodomain of a co-stimulatory molecule, preferably an endodomain of OX40. In some embodiments, the endodomain of OX40 comprises the amino acid sequence of SEQ ID NO:11.

In some embodiments, the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; and optionally, the α chain comprises an endodomain of OX40 linked to the C-terminus of the constant region (for example, linked by a linker, such as (G4S)n linker, wherein n represents an integer from 1 to 10, preferably, n is 3).

In some embodiments, the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 6 such as E is substituted by D, K at position 13 is substituted by R, the amino acids at positions 15-18 are deleted, the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; optionally, the α chain comprises an endodomain of OX40 linked to the C-terminus of the constant region (for example, linked by a linker, such as (G4S)n linker, wherein n represents an integer from 1 to 10, preferably, n is 3).

In some embodiments, the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; and the modified TCR α chain constant region lacks the endodomain of the constant region, for example, amino acids at positions 136-137 are deleted, as compared to the wild-type mouse TCRα chain constant region; and optionally, the α chain comprises an endodomain of OX40 linked to the C-terminus of the constant region (for example, linked by a linker, such as (G4S)n linker, wherein n represents an integer from 1 to 10, preferably, n is 3).

In some embodiments, the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 6 such as E is substituted by D, K at position 13 is substituted by R, the amino acids at positions 15-18 are deleted, the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; and the modified TCR α chain constant region lacks the endodomain of the constant region, for example, amino acids at positions 136-137 are deleted, as compared to the wild-type mouse TCRα chain constant region; and optionally, the α chain comprises an endodomain of OX40 linked to the C-terminus of the constant region (for example, linked by a linker, such as (G4S)n linker, wherein n represents an integer from 1 to 10, preferably, n is 3).

In some embodiments, the α chain comprises an amino acid sequence shown in SEQ ID NO: 13, 15, 17 or 19.

In some embodiments, the β chain comprises a second antigen binding region against GPC3 and a second constant region, wherein the second antigen binding region against GPC3 comprises the amino acid sequence of the light chain variable region shown in SEQ ID NO: 10; the second constant region is a modified TCRβ chain constant region, which is derived from a mouse TCRβ chain constant region, and the amino acid at position 56 such as serine S is mutated to cysteine C, as compared to the wild type mouse TCRβ chain constant region; and optionally, the β chain comprises an endodomain of OX40 linked to the C-terminus of the constant region (for example, linked by a linker, such as (G4S)n linker, wherein n represents an integer from 1 to 10, preferably, n is 3).

In some embodiments, the β chain comprises a second antigen binding region against GPC3 and a second constant region, wherein the second antigen binding region against GPC3 comprises the amino acid sequence of the light chain variable region shown in SEQ ID NO: 10; the second constant region is a modified TCRβ chain constant region, which is derived from a mouse TCRβ chain constant region, and the amino acid at position 56 such as serine S is mutated to cysteine C, the amino acid at position 3, such as R, is substituted by K, the amino acid at position 6, such as T, is substituted by F, K at position 9 is substituted by E, S at position 11 is substituted by A, L at position 12 is substituted by V, and amino acids at positions 17 and 21-25 are deleted, as compared to the wild type mouse TCRβ chain constant region; and optionally, the β chain comprises an endodomain of OX40 linked to the C-terminus of the constant region (for example, linked by a linker, such as (G4S)n linker, wherein n represents an integer from 1 to 10, preferably, n is 3).

In some embodiments, the β chain comprises a second antigen binding region against GPC3 and a second constant region, wherein the second antigen binding region against GPC3 comprises the amino acid sequence of the light chain variable region shown in SEQ ID NO:10; the second constant region is a modified TCRβ chain constant region, which is derived from a mouse TCRβ chain constant region, and the amino acid at position 56 such as serine S is mutated to cysteine C, as compared to the wild type mouse TCRβ chain constant region; and the modified TCR β chain constant region lacks the endodomain of the constant region, for example, amino acids at positions 167-172 are deleted, as compared to the wild-type mouse TCRβ chain constant region; and optionally, the β chain comprises an endodomain of OX40 linked to the C-terminus of the constant region (for example, linked by a linker, such as (G4S)n linker, wherein n represents an integer from 1 to 10, preferably, n is 3).

In some embodiments, the β chain comprises a second antigen binding region against GPC3 and a second constant region, wherein the second antigen binding region against GPC3 comprises the amino acid sequence of the light chain variable region shown in SEQ ID NO:10; the second constant region is a modified TCRβ chain constant region, which is derived from a mouse TCRβ chain constant region, and the amino acid at position 56 such as serine S is mutated to cysteine C, the amino acid at position 3, such as R, is substituted by K, the amino acid at position 6, such as T, is substituted by F, K at position 9 is substituted by E, S at position 11 is substituted by A, L at position 12 is substituted by V, and amino acids at positions 17 and 21-25 are deleted, as compared to the wild type mouse TCRβ chain constant region; and the modified TCR β chain constant region lacks the endodomain of the constant region, for example, amino acids at positions 167-172 are deleted, as compared to the wild-type mouse TCRβ chain constant region; and optionally, the β chain comprises an endodomain of OX40 linked to the C-terminus of the constant region (for example, linked by a linker, such as (G4S)n linker, wherein n represents an integer from 1 to 10, preferably, n is 3).

In some embodiments, the β chain comprises an amino acid sequence shown in SEQ ID NO: 14, 16, 18 or 20.

In some embodiments, the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; and the modified TCR α chain constant region lacks the endodomain of the constant region, for example, amino acids at positions 136-137 are deleted, as compared to the wild-type mouse TCRα chain constant region; and the α chain comprises an endodomain of OX40 linked to the C-terminus of the constant region (for example, linked by a linker, such as (G4S)n linker, wherein n represents an integer from 1 to 10, preferably, n is 3); and
the β chain comprises a second antigen binding region against GPC3 and a second constant region, wherein the second antigen binding region against GPC3 comprises the amino acid sequence of the light chain variable region shown in SEQ ID NO:10;the second constant region is a modified TCRβ chain constant region, which is derived from a mouse TCRβ chain constant region, and the amino acid at position 56 such as serine S is mutated to cysteine C, as compared to the wild type mouse TCRβ chain constant region.

In some embodiments, the STAR comprises an α chain set forth in SEQ ID NO:13 and a β chain set forth in SEQ ID NO:14. In some embodiments, the STAR comprises an α chain set forth in SEQ ID NO:15 and a β chain set forth in SEQ ID NO:16. In some embodiments, the STAR comprises an α chain set forth in SEQ ID NO:17 and a β chain set forth in SEQ ID NO:18. In some embodiments, the STAR comprises an α chain set forth in SEQ ID NO:19 and a β chain set forth in SEQ ID NO:20. In some embodiments, the STAR comprises an α chain set forth in SEQ ID NO:15 and a β chain set forth in SEQ ID NO:14. In some embodiments, the STAR comprises an α chain set forth in SEQ ID NO:19 and a β chain set forth in SEQ ID NO:18.

In another aspect, the present invention provides a method for preparing the therapeutic T cell according to the present invention, comprising co-expressing CXCR2 and a Synthetic T cell Receptor and Antigen Receptor (STAR) against GPC3 (Glypican 3) as defined above in the present invention in the T cell. In some embodiments, the method comprises introducing into the T cell an expression vector comprising a nucleotide sequence encoding said CXCR2, an expression vector comprising a nucleotide sequence encoding said α chain, and an expression vector comprising a nucleotide sequence encoding said β chain. In some embodiments, the encoding nucleotide sequence is operably linked to a regulatory sequence.

The "expression vector" of the present invention may be a linear nucleic acid fragment, a cyclic plasmid, a viral vector, or an RNA capable of translation (e.g., mRNA). In some preferred embodiments, the expression vector is a viral vector, such as a lentiviral vector.

The term "regulatory sequence" and "regulatory element" are used interchangeably to refer to a nucleotide sequence that is located upstream (5' non-coding sequence), intermediate or downstream (3' non-coding sequence) of a coding sequence and affect the transcription, RNA processing or stability, or translation of the relevant coding sequence. An expression regulatory element refers to a nucleotide sequence that can control the transcription, RNA processing or stability, or translation of a nucleotide sequence of interest. A regulatory sequence may include, but is not limited to, a promoter, a translation leader sequence, an intron, an enhancer, and a polyadenylation recognition sequence. As used herein, the term "operably linked" means that a regulatory element (e.g., but not limited to, a promoter sequence, a transcription termination sequence, etc.) is linked to a nucleic acid sequence (e.g., a coding sequence or an open reading frame) such that the nucleotide sequence transcription is controlled and regulated by the transcriptional regulatory element. Techniques for operably linking a regulatory element region to a nucleic acid molecule are known in the art.

In some embodiments, the expression vector comprising the nucleotide sequence encoding the CXCR2, the expression vector comprising the nucleotide sequence encoding the α chain, and the expression vector comprising the nucleotide sequence encoding the β chain may be different expression vectors or may be a same expression vector. For example, the three coding nucleotide sequences may be located respectively in three different expression vectors; or two of them are located in a same expression vector, while the other is located in another expression vector; or all are located in a same expression vector.

In some embodiments, the nucleotide sequence encoding the α-chain and the nucleotide sequence encoding the β-chain may be located in a same expression vector in frame, and may contain a nucleotide sequence encoding a self-cleaving peptide therebetween. The nucleotide sequence encoding the α chain may be located at the 5' end or 3' end of the nucleotide sequence encoding the β chain.

As used herein, the "self-cleavage peptide" means a peptide that can achieve self-cleavage in a cell. For example, the self-cleavage peptide may contain a protease recognition site so as to be recognized and specifically cleaved by proteases in a cell. Alternatively, the self-cleavage peptide may be a 2A polypeptide. The 2A polypeptide is a kind of short peptide from virus, and its self-cleavage occurs during translation. When two different target proteins are linked by 2A polypeptide and expressed in the same reading frame, the two target proteins are generated almost in a ratio of 1:1. A common 2A polypeptide may be P2A from porcine techovirus-1, T2A from Thosea asigna virus, E2A from equine rhinitis A virus, and F2A from foot-and-mouth disease virus. Among them, P2A has the highest cleavage efficiency and is therefore preferred. A variety of functional variants of these 2A polypeptides are also known in the art, which can also be used in the present invention.

In some further embodiments, the expression vector comprising the nucleotide sequence encoding the α chain and the nucleotide sequence encoding the β chain may further comprise a nucleotide sequence encoding the CXCR2, wherein the nucleotide sequence encoding the CXCR2 may be separated from the nucleotide sequence encoding the α chain and the nucleotide sequence encoding the β chain by an IRES (internal ribosome entry site) sequence.

The T cell of the present invention may be obtained by various non-limiting methods from a number of non-limiting sources, including peripheral blood mononuclear cells, bone marrow, lymph node tissue, cord blood, thymus tissue, ascite, pleural effusion, spleen tissue, and tumor. In some embodiments, the cell may be derived from a healthy donor or from a patient diagnosed as cancer. In some embodiments, the cell may be part of a mixed population of cells showing distinct phenotypic profiles. For example, T cells can be obtained by isolating peripheral blood mononuclear cells (PBMC), then activating and amplifying with a specific antibody.

In some embodiments of aspects of the invention, the T cells are derived from autologous cells of a subject. As used herein, "autologous" means that cells, cell line, or population of cells used to treat a subject is derived from the subject. In some embodiments, the immune cells, such as T cells, are derived from allogeneic cells, such as a donor compatible with the subject human leukocyte antigen (HLA). Cells from donors can be converted into non-alloreactive cells using standard protocols and replicated as needed to produce cells that can be administered to one or more patients.

In another aspect, the present invention provides a pharmaceutical composition, which comprises the therapeutic T cell of the present invention and a pharmaceutically acceptable carrier.

As used herein, a "pharmaceutically acceptable carrier" includes any and all physiologically compatible solvents, dispersion medium, coatings, antibacterial and antifungal agents, isotonic agents and absorption retarders, etc. Preferably, the carrier is suitable for intravenous, intramuscular, subcutaneous, parenteral, spinal, or epidermal administration (e.g., by injection or infusion).

In another aspect, the present invention provides the use of the therapeutic T cell of the present invention in the preparation of a medicine for the treatment of a disease such as cancer in a subject.

As use herein, "subject" refers to an organism that suffers from or is prone to suffer from a disease (e.g., cancer) that can be treated by the cell, method, or pharmaceutical composition of the present invention. A non-limiting example includes human, cattle, rat, mouse, dog, monkey, goat, sheep, cow, deer, and other non-mammals. In some preferred embodiments, the subject is human.

In another aspect, the present invention provides a method for treating a disease such as cancer in a subject, the method comprising administering to the subject an effective amount of therapeutic immune cell or pharmaceutical composition of the present invention.

As used herein, a "therapeutically effective amount" or "therapeutically effective dose" or "effective amount" refers to the amount of a substance, compound, material or cell that is at least sufficient to produce a therapeutic effect after administration to a subject. Therefore, it is an amount necessary to prevent, cure, improve, block or partially block the symptoms of disease or disorder. For example, an "effective amount" of the cell or pharmaceutical composition of the present invention may preferably result in a decrease in the severity of disorder symptoms, an increase in the frequency and duration of the asymptomatic period of the disorder, or the prevention of injury or disability as a result of suffering from the disorder. For example, for the treatment of tumor, an "effective amount" of the cell or pharmaceutical composition of the present invention may preferably inhibit tumor cell growth or tumor growth by at least about 10%, preferably at least about 20%, more preferably at least about 30%, more preferably at least about 40%, more preferably at least about 50%, more preferably at least about 60%, more preferably at least about 70%, and more preferably at least about 80%, as compared to an untreated subject. The ability to inhibit tumor growth can be evaluated in an animal model system that may predict efficacy in a human tumor. Alternatively, it is possible to perform evaluation by examining the ability to inhibit the growth of tumor cells which may be determined in vitro by tests known to those skilled in the art.

In practice, the dose level of cells in the pharmaceutical composition of the present invention may vary to obtain an amount of the active ingredient that can effectively achieve the desired therapeutic response to a specific patient, composition and administration route without toxicity to the patient. The chosen dose level depends on a variety of pharmacokinetic factors, including the activity of the applied particular composition of the invention, administration route, administration time, excretion rate of the applied particular compound , duration of treatment, applied other drugs, compounds and/or materials in combination with the applied particular composition, age, gender, weight, condition, general health and medical history of the patient to be treated, and similar factors known in the medical field.

The administration of the therapeutic T cell or pharmaceutical composition or drug according to the present invention may be carried out in any convenient manner, such as through injection, infusion, implantation or transplantation. The administration of the cell or composition described herein may be intravenous, intralymphatic, intradermal, intratumoral, intramedullary, intramuscular, or intraperitoneal administration. In one embodiment, the cell or composition of the present invention is preferably administered by intravenous injection.

In embodiments of various aspects of the present invention, the disease is a GPC3-related disease, such as a disease related to abnormal expression of GPC3, such as a GPC3-related cancer. The cancer is for example liver cancer such as hepatocellular carcinoma, lung cancer such as lung squamous cell carcinoma (SqCC), gastric cancer, ovarian cancer, melanoma or pediatric embryonal tumor.

### Example

### Experimental materials and methods

### Vector construction

The lentiviral vectors and lentiviral packaging plasmids used in the examples of this application were purchased from commercial companies or synthesized by commercial companies. The gene fragments used in the examples of this application, including signal peptides, antibody binding regions, hinge regions, TCR constant regions, tag proteins, etc., were synthesized from commercial companies. By means of PCR with synthetic primers, one or more target fragments were linked to obtain corresponding functional sequences. The lentiviral vector used in the present invention is pHAGE-EF1α-RFP. The pHAGE-EF1A-WPRE-AMP vector was obtained using restriction endonuclease Not I/Cla I. The fragment genes were obtained by synthesis and PCR method. Then the complete vector was obtained by homologous recombination method under the action of recombinase.

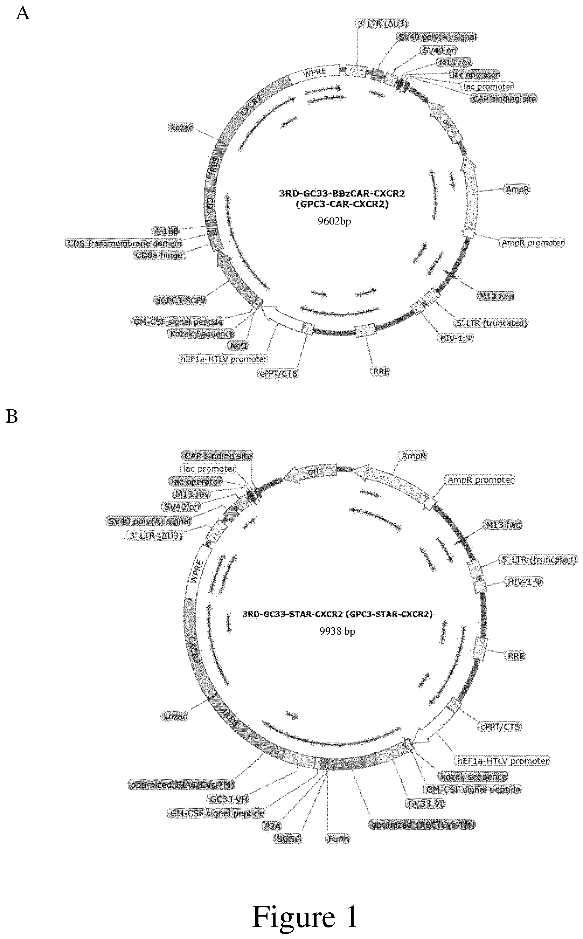

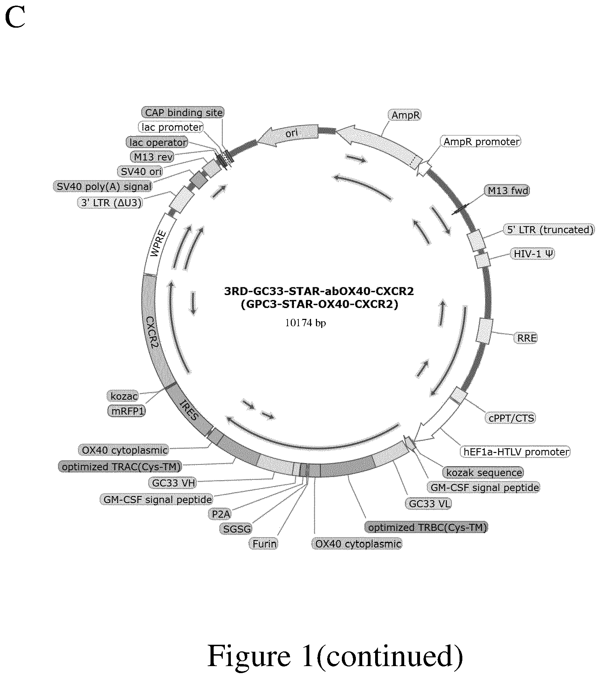

The following constructs were constructed in the examples of the present application: 3RD-GC33-BBzCAR-CXCR2 (GPC3 CAR-CXCR2), and the constructs 3RD-GC33-STAR-abOX40-CXCR2 (GPC3 STAR-OX40-CXCR2) and 3RD-GC33-STAR-CXCR2 (GPC3 STAR-CXCR2) derived from TRAC (Cys-TM) and TRBC (Cys-TM). Maps of the constructs are shown in Figure 1. Maps of constructs using TRAC (Nrec-Cys-TM) and TRBC (Nrec-Cys-TM) are not shown.

The amino acid sequence of GPC3 CAR-CXCR2 as a control is shown in SEQ ID NO:21.

The antigen-binding region targeting GPC3 is derived from antibody GC33, the amino acid sequence of its heavy chain variable region (VH) is shown in SEQ ID NO:9; the amino acid sequence of its light chain variable region (VL) is shown in SEQ ID NO:10 .

The constant region of the α chain is derived from mouse, the wild type is named TRAC and the sequence thereof is shown in SEQ ID NO:2. TRAC(Cys-TM) refers to the murine constant region with the threonine T at position 48 mutated to cysteine C and the mutation from LSVMGLRIL to LLVIVLRIL. The amino acid sequence of TRAC(Cys-TM) is shown in SEQ ID NO:3. TRAC (Nrec-Cys-TM) refers to the murine constant region with amino acid at position 6 such as E being substituted by D, K at position 13 substituted by R, amino acids at positions 15-18 deleted, and threonine T at position 48 mutated to cystine C and the sequence LSVMGLRIL mutated to LLVIVLRIL, the amino acid sequence of TRAC (Nrec-Cys-TM) is shown in SEQ ID NO:4.

The constant region of the β chain is derived from mouse, the wild type is named TRBC and the sequence thereof is shown in SEQ ID NO:6. TRBC (Cys-TM) refers to the murine constant region in which serine S at position 56 is mutated to cysteine C, and its amino acid sequence is shown in SEQ ID NO:7. TRBC (Nrec-Cys-TM) refers to the murine constant region in which the amino acid at position 56 such as serine S is mutated to cysteine C, the amino acid at position 3 such as R is substituted by K, and the amino acid at position 6 such as T is substituted by F , K at position 9 is substituted by E, S at position 11 is substituted by A, L at position 12 is substituted by V, and amino acids 17, 21-25 are deleted, and its amino acid sequence is shown in SEQ ID NO:7 .

The amino acid sequence of the cytoplasmic region of costimulatory molecule OX40 is shown in SEQ ID NO:11. The cytoplasmic region of the co-stimulatory molecule OX40 is linked to the C-terminus of the α chain constant region (based on TRAC (Nrec-Cys-TM) or TRAC (Cys-TM)) lacking the endodomain and the C-terminus of the β chain constant region (based on TRBC (Nrec-Cys-TM) or TRBC(Cys-TM)) lacking the endodomain, through the (G₄S)₃ linker.

### Lentiviral packaging

Lentix-293T cells were inoculated into 10 cm culture dishes at 5×10⁵ cells/mL, cultured in a 37°C, 5% CO₂ incubator, and transfected when the cell density reached about 80% (observed under a microscope). The three plasmids were mixed evenly with 500uL serum-free DMEM according to the ratio of PMD2.G :PSPAX:transfer plamid=1:2:3. 54uL PEI-max was mixed with 500uL serum-free DMEM evenly, and placed at room temperature for 5min (the volume-mass ratio of PEI-Max and plasmid was 3:1). The PEI-max mixture was slowly added to the plasmid mixture, gently pipetted, mixed well, and placed at room temperature for 15 minutes. The final mixture was slowly added into the culture medium, mixed well, put back into the incubator for culturing for 12h-16h, then changed to 6% FBS DMEM medium for culturing, and the virus liquid was collected at 48 h and 72 h.

### Culture and infection of T cells

### 1) Culture of Jurkat T cell line

The Jurkat T cell line was cultured in RPMI 1640 medium containing 10% FBS. The culture density was 3*10⁵/ml, with the highest density not more than 3*10⁶/ml. The cells were subcultured every 1-2 days. The required amount of cells were taken after counting the cells, and the medium was added to adjust to the above density, and cultured in a CO₂ incubator .

### 2) Infection of Jurkat T cell line

After cell counting, 1*10⁶/ml cells were centrifuged to replace the medium, resuspended with 1ml RPMI 1640 medium containing 10% FBS, added to a 24-well plate, and virus solution was added (MOI=1), centrifuged at 1500 rpm for 90 min, placed in a CO₂ incubator. After 12 hours of infection, the medium was completely changed to fresh RPMI 1640 medium containing 10% FBS, and the positive rate was detected within 72 hours.

### 3) Culture of human primary T cells

After the primary T cells were obtained by Ficoll separation method, they were cultured in X-VIVO medium containing 10% FBS and 100 IU/ml IL-2. The initial culture density was 1*10⁶/ml. The cells were added to a CD3 and RetroNectin (final concentration 5 µg/ml) pre-coated plate. During the later stage, the culture density was 5*10⁵/ml, with the highest not more than 3*10⁶/ml, and the cells were subcultured every 1-2 days.

### 4) Infection of human primary T cells

After the primary T cells were cultured for 48 hours, the virus solution was added, MOI=20, centrifuged at 1500 rpm for 90 minutes, and placed in a CO₂ incubator for culture. After 24 hours of infection, X-VIVO medium containing 10% FBS and 100 IU/ml IL-2 was supplemented and transferred to another well, and the infection efficiency was detected by tag protein or antibody at 72 hours.

### 5) Detection of infection efficiency

After 72 hours of infection, the cells were blown evenly and counted, and 5*10⁵/ml was taken for centrifugation, and the supernatant was discarded. The staining solution was PBS+2% FBS+2 mM EDTA, and the corresponding antibody was added, incubated for 30 minutes, and then washed twice with PBS, detected by the machine.

### Test of the in vitro function of STAR-T cells expressing CXCR2

Positive T cells were co-cultured with Luciferase-expressing target cells (SK-Hep-1 with low expression of GPC3, Huh-7 with medium expression of GPC3, HepG2 with high expression of GPC3) at specified effect-to-target ratios.

After co-cultivation for 24 h, the cell suspension was gently blown evenly, and 150 µL of the cell suspension of each well was added to a white 96-well plate, each with two replicate wells, and luciferase substrate was added. The mixture was shaken (at low speed) and incubated for 10 min, then a multifunctional microplate reader was used to detect the chemiluminescence value. Calculation of cell killing: killing efficiency=100%-(value of the effector cell-target cell well / value of the control cell-target cell well). Alternatively, after 24 hours of co-cultivation, the cell culture supernatant was collected, and the content of cytokines was detected by a commercial kit.

### Test of the in vivo function of STAR-T cells expressing CXCR2

1. Establishment of mouse tumor model
(1) 10 NCG mice with an age of 5-6 weeks and a weight of about 20 g were purchased and raised in the animal room for 3-5 days.
(2) Matrigel was thawed on ice 3-5 hours. And PBS, pipette tips, etc were pre-cooled.
(3) HuH7-Luc/GFP cells were washed twice with PBS and counted, divided into 5 groups and diluted to 1×10°/mL, 5×10⁶/mL, 1×10⁷/mL, 5 ×10⁷/mL, 1×10⁸/mL, respectively, 250 µL for each group was prepared.
(4) The melted Matrigel was added to each group of cells on ice, 250 µL/group, gently blown and mixed with a pipette (pre-cooled pipette tip), PBS was added to 500 µL.
(5) Everything (cells, syringes, pipettes, tips) was put on ice, sterilized and brought into the animal room for tumor inoculation.
(6) The mice were anesthetized with an isoflurane gas anesthesia apparatus, and divided into 5 groups, two mice in each group. Tumor cells were inoculated subcutaneously on the right back of the mouse, 100 µL/mouse. This day was recorded as Day 0.
(7) On Day 3, Day 6, Day 9, and Day 12, the tumors of the mice were measured and detected by live imaging. The appropriate tumor inoculation dose and growth time were selected for subsequent experiments.

2. Analysis of tumor killing function of T cells in vivo
(1) Corresponding number of mice was set up according to the experimental groups, 5-6 mice in each group. T cells were infected and positive cell were sorted in advance, and cultured for 7-10 days before reinfusion.
(2) Tumor cells were inoculated according to the tumor inoculation amount obtained in the preliminary experiment, 3×10⁶ HuH-7-Luc/GFP cells per mouse.
(3) When the tumor size growed to about 100 cm³ (7 days), in vivo fluorescence imaging was performed, and the mice were divided into several groups according to the tumor size, and T cells were reinfused into the tail vein, including control T cells, traditional STAR-T cells and improved STAR-T cells. The amount of reinfusion per mouse was 1×10⁶-3×10⁶ positive T cells.
(4) Every 3-4 days after reinfusion, the size of the tumor was detected with an in vivo imager, and the peripheral blood of the mouse was collected to analyze the proliferation of T cells in vivo.
(5) After 35 days of tumor inoculation, the mice in each group were killed, the tumors were stripped, and the peripheral blood, spleen and other tissues of the mice were collected to analyze the distribution of T cells, type of T cells, and exhaustion of tumor-infiltrating cells.

3. In vivo imaging of mice
After the mice were anesthetized with isoflurane, they were weighed and recorded, and 200 µL of D-fluorescein potassium salt was injected intraperitoneally. After 5-7 minutes, the mice were placed in an in vivo imager for imaging. The imaging time was 1 min. (Note: Imaging time is determined by tumor type and size but needs to be consistent throughout the experiment). After imaging, the imaging photos were saved, the tumor area was circled, and the total fluorescence value of the tumor area of each mouse was record.
4. In vivo T cell migration analysis
(1) 36 mice were purchased. T cells were infected and positive cells were sorted in advance, and cultured for 7-10 days before reinfusion.
(2) Tumor cells were inoculated, 3.5×10⁶ HuH-7-Luc/GFP cells per mouse.
(3) When the tumor size grown to about 100cm³ (7 days), the in vivo fluorescence imaging was performed, and the mice were divided into three groups according to the tumor size, 12 mice in each group, and T cells were reinfused into the tail vein, including control T cells, traditional STAR-T cells and novel CXCR2-modified STAR-T cells. The reinfusion amount of each mouse was 2×10⁶ positive T cells.
(4) On Day 1, Day 3, Day 5 and Day 7 after T cell reinfusion, mice were taken to analyze tumor infiltrating cells. Three mice in each group were sacrificed, the tumors were peeled off, and the weight of the tumors was weighed. Half of the tumor was excised and fixed in 4% PFA for immunohistochemical staining.
(5) The remaining 1/2 tumor were weighed, chopped up, ground, and washed twice with PBS, then staining solution was added (see the table below for the staining protocol), 60 µL/sample, and transferred to a 96-well plate for staining, and stained on ice for 30 min in the dark.
Protocol for T cell staining in mouse tumors

| Antibody | fluorescent label | Dilution ratio |
|---|---|---|
| Mouse CD45.2 | PerCP/Cy5.5 | 1:200 |
| Human CD45 | BV421 | 1:200 |
| Human CD3 | PE/Cy7 | 1:200 |

(6) The cells were centrifuged at 1800 rpm for 5 minutes at 4°C, the supernatant was discarded, and the cells were washed twice with 200 µL of PBS.
(7) The cells were resuspended after adding 200 µL of 4% PFA, filtered through a 200-mesh filter into a flow tube, put in a 4°C refrigerator, and tested on a machine within 24 hours. The number of infiltration T cells per unit weight of tumor tissue was calculated according to the count and tumor weight.

### Example 1. Optimization of STAR structure

In the present invention, murineization of the constant region, cysteine point mutation, , and hydrophobic amino acid mutation of the constant region of the α chain improve the function of the STAR.

Constant region murinization modification: since the constant region sequences of human, primate and mouse TCRα/β chains have high functional conservation and the key amino acid sequences are the same, they can be exchanged with each other. After the exchange, on the one hand, the efficiency of correct pairing of STAR molecules is increased, and on the other hand, the possibility of unknown specificity caused by mismatching is reduced, which increases safety.

Cysteine point mutation to introduce disulfide bond: threonine T at position 48 in the constant region of the murineized TCRα chain was mutated to cysteine C, and serine S at position 56 in the constant region of the murineized TCRβ chain was mutated to cysteine C. These two newly added cysteines would form a disulfide bond between the two chains of STAR, reduce the mismatch between the two chains of STAR and the endogenous TCR chain, and help STAR molecules form a more stable complex.

The design of hydrophobic amino acid substitution in the transmembrane region of STAR: three amino acid sites in the transmembrane region of the constant region of the TCRα chain from 111 to 119 were mutated, serine S at position 112 mutated to leucine L and Methionine M at position 114mutated to isoleucine I, glycine G at position 115 mutated to valine V. The overall amino acid sequence of this region was mutated from LSVMGLRIL to LLVIVLRIL. This design increases the hydrophobicity of the transmembrane region, reduces the instability caused by the positive charge carried by the TCR transmembrane region, and enables STAR molecules to exist more stably on the cell membrane, thereby obtaining better functions.

In order to further optimize the design of the STAR molecule, specific rearrangement of the N-terminus of the constant region of the STAR molecule was carried out on the basis of murineization of the constant region, cysteine point mutation and hydrophobic amino acid mutation of the α-chain constant region to obtain better effects. Rearrangement means to delete part of the sequence and at the same time carry out humanization mutation on part of the sequence. The significance of humanized mutations is to reduce the non-human sequences in STAR molecules as much as possible while ensuring the function of STAR molecules, so as to avoid the possibility of STAR-T cells being rejected by receptors in clinical applications to the greatest extent.

To this end, the 18 amino acids at the N-terminal of the constant region of the TCRα chain were further modified, including amino acids at position 6 such as E being replaced by D, K at position 13 being replaced by R, and amino acids at positions 15-18 being deleted. The obtained constant region of the α-chain was named TRAC (Nrec-Cys-TM). The 25 amino acids at the N-terminal of the TCRβ chain constant region were further modified, including the amino acid at position 3 such as R being replaced by K, the amino acid at position 6 such as T being replaced by F, K at position 9 being replaced by E, and the amino acid S at position 11 being replaced by A, the L at position 12 being replaced by V, and amino acids at positions 17 and 21-25 being deleted, and the resulting β-chain constant region was named TRBC (Nrec-Cys-TM).

In addition, co-stimulatory molecules such as the cytoplasmic region of OX40 can be linked to the C-terminus of the α-chain constant region and/or the C-terminus of the β-chain constant region to further enhance the function of STAR. The co-stimulatory molecule may be linked to the C-terminus of the α-chain constant region and/or the C-terminus of the β-chain constant region via a linker, e.g., to a (G4S)₃ linker. In addition to the above-mentioned modifications, the constant region linked to the co-stimulatory molecule can also lack the natural endodomain relative to the wild-type constant region, which further improves the function of the STAR. For example, amino acids 136-137 may be deleted from the α chain constant region; and/or amino acids 167-172 may be deleted from the βchain constant region.

### Example 2. Comparison of in vitro target killing ability and cytokine secretion ability of GPC3-targeting CAR-T cells co-expressing CXCR2 and GPC3-targeting STAR-T cells co-expressing CXCR2

A GPC3-targeting CAR was constructed using the scFv of the antibody GC33 against GPC3. The CAR contains a CD8a hinge region, a CD8 transmembrane region, a 41-BB co-stimulatory domain, and a CD3 signaling domain.

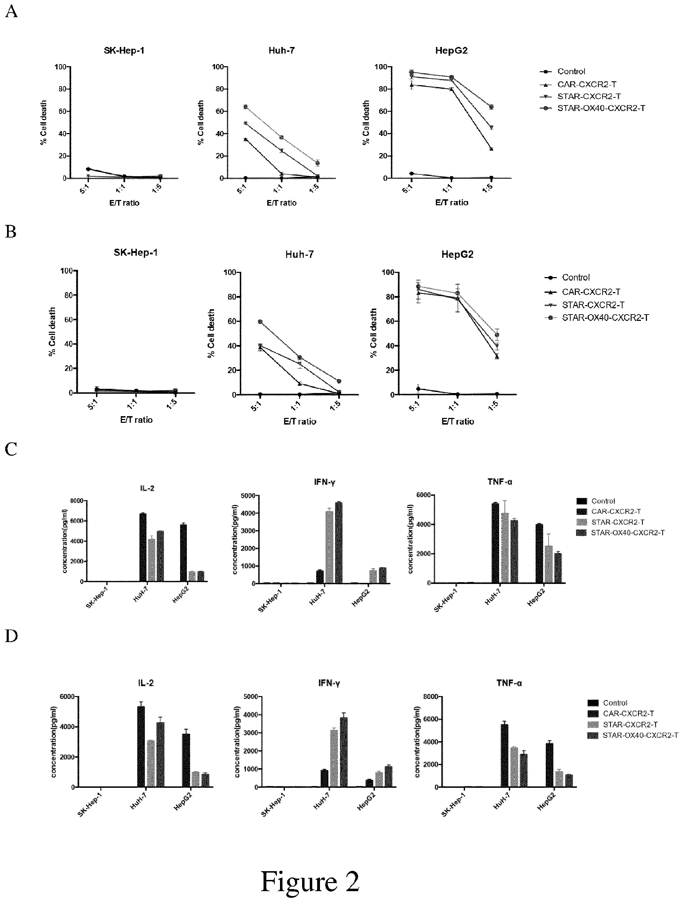

STARs targeting GPC3 were constructed based on the optimized constant regions in Example 1 using the light chain and heavy chain variable regions of antibody GC33 (constant region in Figure 2 A and C: Nrec-Cys-TM; constant region in Figure 2 B and D: Cys-TM).

The obtained CAR and STAR structures were co-expressed with CXCR2 in T cells to obtain corresponding GPC3 CAR-CXCR2 T cells and GPC3 STAR-CXCR2 T cells.

Then, the target killing effect of the obtained T cells was tested using liver cancer cell lines expressing different degrees of GPC3 (SK-Hep-1 with low GPC3 expression, Huh-7 with medium GPC3 expression, and HepG2 with high GPC3 expression). The results are shown in Figure 2. In the case of expressing CXCR2, GPC3 STAR had higher target cell killing ability and IFNγ secretion ability, and the effect of GPC3 STAR with OX40 co-stimulatory domain was the best.

### Example 3. Comparison of in vivo tumor infiltration and tumor suppression capabilities of GPC3-targeting CAR-T cells co-expressing CXCR2 and GPC3-targeting STAR-T cells (with optimized Nrec-Cys-TM constant region structure) co-expressing CXCR2

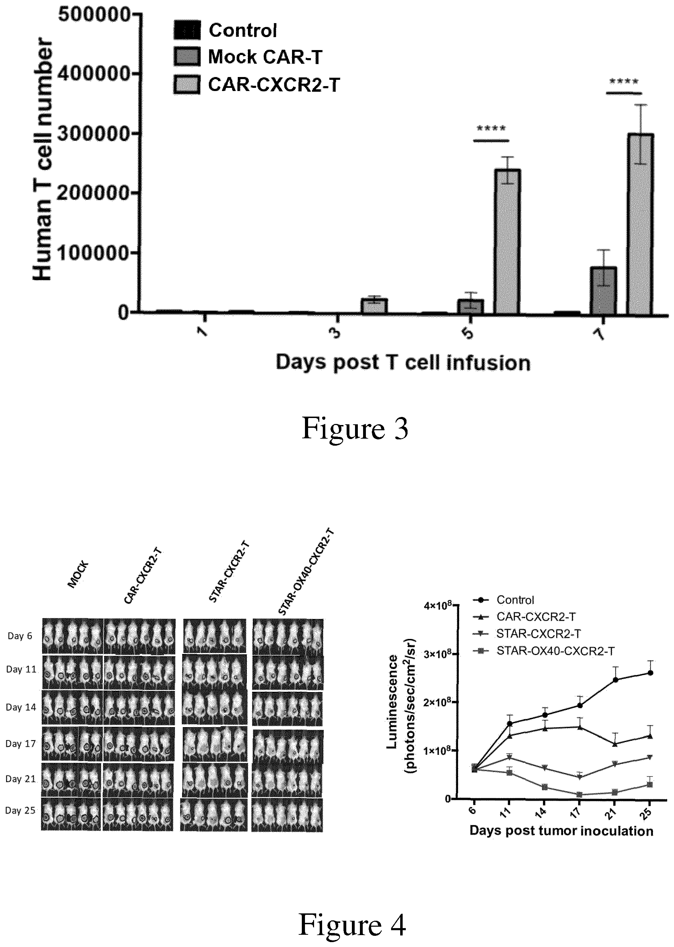

Tumor model was established by inoculating mice with HuH7-Luc/GFP cells, and the in vivo tumor infiltration and tumor suppression capabilities of different therapeutic T cells were tested. The experimental results are shown in Figure 3, CXCR2 co-expression can significantly improve the infiltration of therapeutic CAR-T cells into tumors. As shown in Figure 4, in the case of expressing CXCR2, GPC3-STAR had a higher tumor suppressive ability, and the GPC3-STAR (with optimized Nrec-Cys-TM constant region structure) with OX40 costimulatory domain had the best effect. The STAR described in this experiment was derived from TRAC (Nrec-Cys-TM) and TRBC (Nrec-Cys-TM).

### Example 4. In vivo and in vitro efficacy analysis of GPC3-targeted STAR-T cells co-expressing CXCR2 (with optimized structure of Cys-TM constant region and only α-chain added with co-stimulatory domain)

In this example, in vitro and in vivo efficacy of the STAR-T cells co-expressing CXCR2 based on the optimized structure of the Cys-TM constant region and in which only the α chain was added with the OX40 co-stimulatory domain (OX40 was added to the Cys-TM constant region of the α chain with the deletion of the endodomain) was analyzed..

### 1. Compared with the corresponding GPC3 STAR-aOX40-T that does not co-express CXCR2, GPC3 STAR-aOX40-CXCR2-T (Cys-TM) has better in vitro function and migration ability

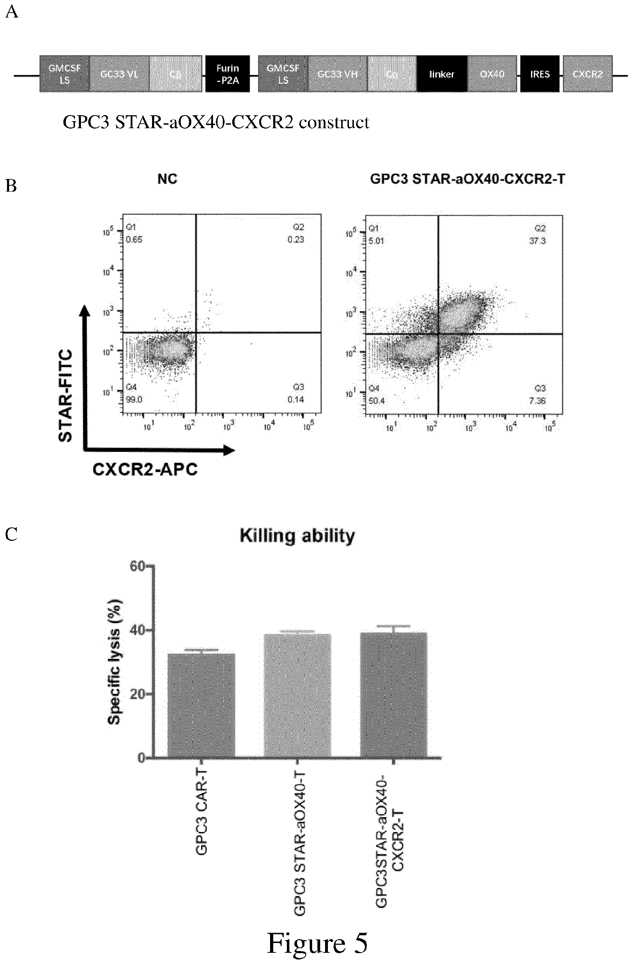

The construction strategy of GPC3 STAR-aOX40-CXCR2-T(Cys-TM) is shown in Figure 5A. The STAR was derived from TRAC (Cys-TM) and TRBC (Cys-TM).

First, the expression level of CXCR2 on GPC3 STAR-aOX40-CXCR2-T (Cys-TM) cells was detected by flow cytometry. The results are shown in Figure 5B. On T cells infected with virus, mTCRβ antibody staining detected the STAR receptor on the cell membrane, and CXCR2-APC antibody detected the expression of CXCR2 receptor, confirming that GPC3 STAR-aOX40-CXCR2-T(Cys-TM) had good co-expression levels of GPC3-STAR and CXCR2.

Subsequently, GPC3-CAR-T, GPC3 STAR-aOX40-T(Cys-TM) and GPC3 STAR-aOX40-CXCR2-T(Cys-TM) cells were co-cultured respectively with Huh-7 cells at a (1:5) effect-to-target ratio. The killing levels of the three T cells on the target cells were detected after 24 hours. The results are shown in Figure 5C. The results showed that GPC3 STAR-aOX40-CXCR2-T (Cys-TM) had higher killing activity than GPC3 STAR-aOX40-T (Cys-TM) and GPC3 CAR-T.

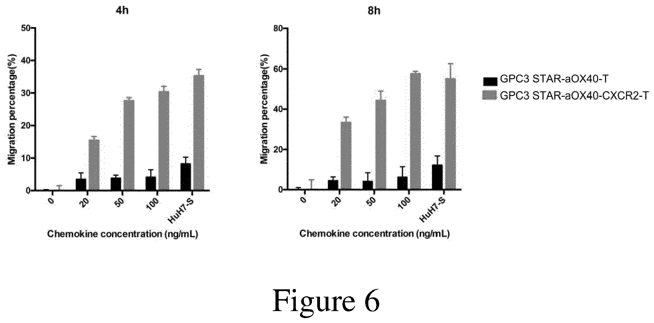

Then GPC3 STAR-aOX40-T (Cys-TM) and GPC3 STAR-aOX40-CXCR2-T (Cys-TM) cell chemotactic movement was detected by Transwell. 200 µL of complete medium containing 1E5 GPC3 STAR-aOX40-T (Cys-TM) and GPC3 STAR-aOX40-CXCR2-T (Cys-TM) was added to the upper chamber of Tranwell, 600 µL of medium was added to the lower chamber in which the indicated gradient concentrations of CXCR2 ligand chemokines were added; another lower layer was set as the culture supernatant group of the liver cancer tumor cell line Huh-7, and the culture medium only was used as the control. After incubating for the specified time, the culture medium in the lower layer was collected to detect the number of cells migrating to the lower layer on a flow cytometer, and the proportion of migrating cells was calculated. The results are shown in Figure 6. The migration ability of GPC3 STAR-aOX40-CXCR2-T (Cys-TM) was positively correlated with the concentration and time of chemokines; GPC3-STAR-aOX40-T (Cys-TM) basically maintained a low migration ability .

### 2. Compared with GPC3 STAR-aOX40-T (Cys-TM), GPC3 STAR-aOX40-CXCR2-T (Cys-TM) has improved tumor directional migration ability in vivo

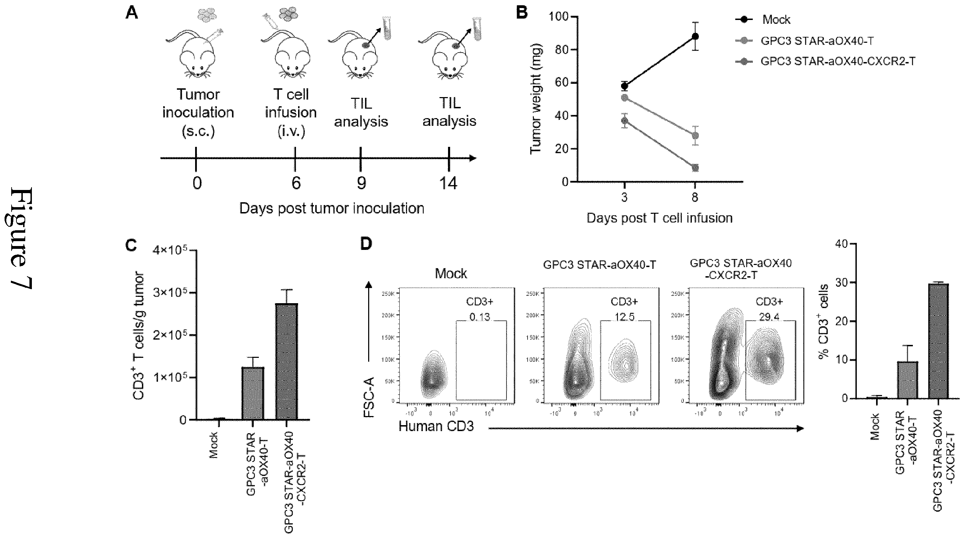

The experimental protocol is shown in Figure 7A. NSG mice were subcutaneously inoculated with HuH-7 tumor cells, and on day 6 after tumor formation, Mock T, GPC3 STAR-aOX40-T (Cys-TM) and GPC3 STAR-aOX40-CXCR2-T (Cys-TM) cells were reinfused via tail vein. Then, on Day 9 and Day 14 after tumor formation, tumor tissues were collected respectively, and the number and proportion of tumor infiltrating T cells were detected.

Figure 7B shows the comparison of tumor tissue weights in Mock T, GPC3 STAR-aOX40-T (Cys-TM) and GPC3 STAR-aOX40-CXCR2-T (Cys-TM) cell reinfusion groups. On Day 3 and Day 8 after the reinfusion of T cells, the tumor-bearing mice were euthanized, and the tumor tissues were taken out, weighed and recorded. The results show that GPC3 STAR-aOX40-CXCR2-T(Cys-TM) could effectively eliminate tumors, and compared with GPC3 STAR-aOX40-T(Cys-TM), the tumor killing speed was faster.

Figure 7C shows a comparison of infiltrating T cells in tumor tissues. The weighed tumor tissue was ground into single cell suspension, and the number of T cells in the tumor tissue was detected on a flow cytometer after antibody staining. Figure 7D shows the proportion of infiltrating CD3+ T cells in tumor tissue detected by flow cytometry (left panel) and quantitative statistical analysis (right panel). The results show that 3 days after T cell reinfusion, GPC3 STAR-aOX40-CXCR2-T(Cys-TM) cells showed a higher number and proportion of tumor infiltration.

### 3. The anti-tumor activity of GPC3 STAR-aOX40-CXCR2-T(Cys-TM) is stronger than that of GPC3 STAR-aOX40-T(Cys-TM)

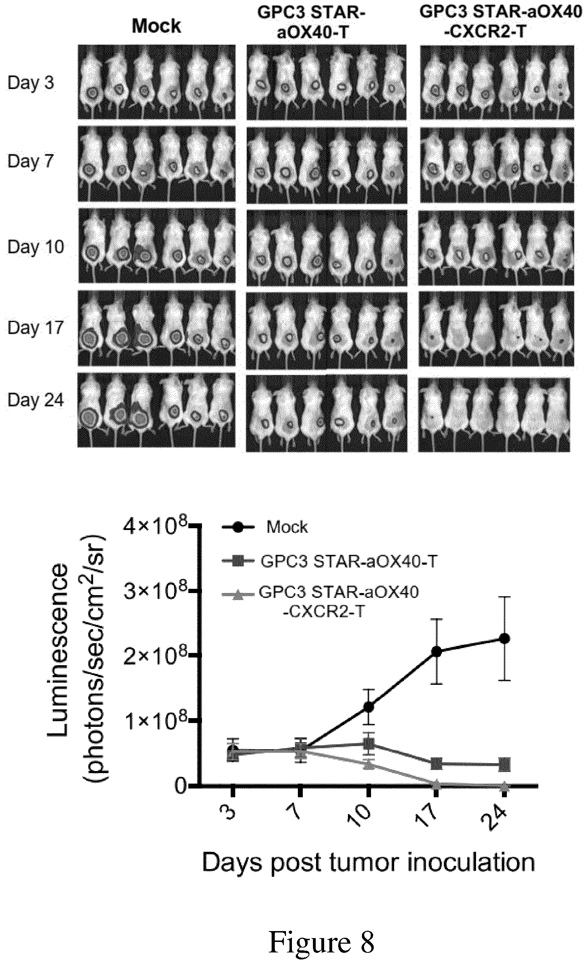

In this experiment, tumor growth was detected by catalyzed luminescence with a luciferin substrate. The HuH-7 cell line stably expressing luciferase was injected subcutaneously into NSG mice, and after 7 days of tumor formation, Mock T, GPC3 STAR-aOX40-T (Cys-TM) and GPC3 STAR-aOX40-CXCR2- T(Cys-TM) were reinfused via tail vein, followed by periodic detection of tumor growth by luciferin substrate catalyzed luminescence. The statistics of the tumor size reflected by the fluorescence value are shown in the right panel of Figure 8. The results show that at a lower reinfusion dose, mice reinfused with GPC3 STAR-aOX40-CXCR2-T(Cys-TM) cells could basically achieve tumor-free. Compared with GPC3 STAR-aOX40-T(Cys-TM), GPC3 STAR-aOX40-CXCR2-T(Cys-TM) had faster and stronger tumor killing efficacy.

Sequence listing:
SEQ ID NO:1 Human T cell receptor alpha chain constant region
DIQNPDPAVYQLRDSKSSDKSVCLFTDFDSQTNVSQSKDSDVYITDKTVLDMRSMDFKSNSAVAWS
NKSDFACANAFNNSIIPEDTFFPSPESSCDVKLVEKSFETDTNLNFQNLSVIGFRILLLKVAGFNLLM
TLRLWSS

SEQ ID NO:2 mouse T cell receptor alpha chain constant region
DIQNPEPAVYQLKDPRSQDSTLCLFTDFDSQINVPKTMESGTFITDKTVLDMKAMDSKSNGAIAWS
NQTSFTCQDIFKETNATYPSSDVPCDATLTEKSFETDMNLNFQNLSVMGLRILLLKVAGFNLLMTLR
LWSS

SEQ ID NO:3 mouse T cell receptor alpha chain constant region containing cysteine substitution and
modification of hydrophobic region (TRAC-Cys-TM)
DIQNPEPAVYQLKDPRSQDSTLCLFTDFDSQINVPKTMESGTFITDKCVLDMKAMDSKSNGAIAWS
NQTSFTCQDIFKETNATYPSSDVPCDATLTEKSFETDMNLNFQNLLVIVLRILLLKVAGFNLLMTLRL
WSS

SEQ ID NO:4 mouse T cell receptor alpha chain constant region containing N-terminal modification,
cysteine substitution and modification of transmembrane hydrophobic region (TRAC-Nrec-Cys-TM)
DIQNPDPAVYQLRDDSTLCLFTDFDSQINVPKTMESGTFITDKCVLDMKAMDSKSNGAIAWSNQTS
FTCQDIFKETNATYPSSDVPCDATLTEKSFETDMNLNFQNLLVIVLRILLLKVAGFNLLMTLRLWSS

SEQ ID NO:5 Human T cell receptor beta chain constant region
DLKNVFPPEVAVFEPSEAEISHTQKATLVCLATGFYPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADC
GFTSESYQQGVLSATILYEILLGKATLYAVLVSALVLMAMVKRKDF

SEQ ID NO:6 mouse T cell receptor beta chain constant region
DLRNVTPPKVSLFEPSKAEIANKQKATLVCLARGFFPDHVELSWWVNGKEVHSGVSTDPQAYKES
NYSYCLSSRLRVSATFWHNPRNHFRCQVQFHGLSEEDKWPEGSPKPVTQNISAEAWGRADCGITS
ASYQQGVLSATILYEILLGKATLYAVLVSTLVVMAMVKRKNS

SEQ ID NO:7 mouse T cell receptor beta chain constant region containing cysteine substitution
(TRBC-Cys-TM)
DLRNVTPPKVSLFEPSKAEIANKQKATLVCLARGFFPDHVELSWWVNGKEVHSGVCTDPQAYKES
NYSYCLSSRLRVSATFWHNPRNHFRCQVQFHGLSEEDKWPEGSPKPVTQNISAEAWGRADCGITS
ASYQQGVLSATILYEILLGKATLYAVLVSTLVVMAMVKRKNS

SEQ ID NO:8 mouse T cell receptor beta chain constant region containing N-terminal modification,
and cysteine substitution (TRBC-Nrecc-Cys-TM)
DLKNVFPPEVAVFEPSAEIATLVCLARGFFPDHVELSWWVNGKEVHSGVCTDPQAYKESNYSYCLS
SRLRVSATFWHNPRNHFRCQVQFHGLSEEDKWPEGSPKPVTQNISAEAWGRADCGITSASYQQGV
LSATILYEILLGKATLYAVLVSTLVVMAMVKRKNS

SEQ ID NO:9 Anti-GPC3 GC33 VH
QVQLVQSGAEVKKPGASVKVSCKASGYTFTDYEMHWVRQAPGQGLEWMGALDPKTGDTAYSQ
KFKGRVTLTADESTSTAYMELSSLRSEDTAVYYCTRFYSYTYWGQGTLVTVSS

SEQ ID NO:10 Anti-GPC3 GC33 VL
DVVMTQSPLSLPVTPGEPASISCRSSQSLVHSNRNTYLHWYLQKPGQSPQLLIYKVSNRFSGVPDRF
SGSGSGTDFTLKISRVEAEDVGVYYCSQNTHVPPTFGQGTKLEIKR

SEQ ID NO:11 OX40 endodomain
RRDQRLPPDAHKPPGGGSFRTPIQEEQADAHSTLAKI

SEQ ID NO:12 CXCR2 amino acid sequence







SEQ ID NO:13 GPC3 STAR-CXCR2(TRAC-Cys-TM) α chain
QVQLVQSGAEVKKPGASVKVSCKASGYTFTDYEMHWVRQAPGQGLEWMGALDPKTGDTAYSQ
KFKGRVTLTADESTSTAYMELSSLRSEDTAVYYCTRFYSYTYWGQGTLVTVSSDIQNPEPAVYQLK
DPRSQDSTLCLFTDFDSQINVPKTMESGTFITDKCVLDMKAMDSKSNGAIAWSNQTSFTCQDIFKE
TNATYPSSDVPCDATLTEKSFETDMNLNFQNLLVIVLRILLLKVAGFNLLMTLRLWSS

SEQ ID NO:14 GPC3 STAR-CXCR2(TRBC-Cys-TM) β chain
DVVMTQSPLSLPVTPGEPASISCRSSQSLVHSNRNTYLHWYLQKPGQSPQLLIYKVSNRFSGVPDRF
SGSGSGTDFTLKISRVEAEDVGVYYCSQNTHVPPTFGQGTKLEIKREDLRNVTPPKVSLFEPSKAEI
ANKQKATLVCLARGFFPDHVELSWWVNGKEVHSGVCTDPQAYKESNYSYCLSSRLRVSATFWHN
PRNHFRCQVQFHGLSEEDKWPEGSPKPVTQNISAEAWGRADCGITSASYQQGVLSATILYEILLGKA
TLYAVLVSTLVVMAMVKRKNS

SEQ ID NO:15 GPC3 STAR-OX40-CXCR2 (TRAC-Cys-TM) α chain
QVQLVQSGAEVKKPGASVKVSCKASGYTFTDYEMHWVRQAPGQGLEWMGALDPKTGDTAYSQ
KFKGRVTLTADESTSTAYMELSSLRSEDTAVYYCTRFYSYTYWGQGTLVTVSSDIQNPEPAVYQLK
DPRSQDSTLCLFTDFDSQINVPKTMESGTFITDKCVLDMKAMDSKSNGAIAWSNQTSFTCQDIFKE
TNATYPSSDVPCDATLTEKSFETDMNLNFQNLLVIVLRILLLKVAGFNLLMTLRLWGGGGSGGGGS
GGGGSRRDQRLPPDAHKPPGGGSFRTPIQEEQADAHSTLAKI

SEQ ID NO:16          GPC3 STAR-OX40-CXCR2(TCRbC-Cys-TM) β chain
DVVMTQSPLSLPVTPGEPASISCRSSQSLVHSNRNTYLHWYLQKPGQSPQLLIYKVSNRFSGVPDRF
SGSGSGTDFTLKISRVEAEDVGVYYCSQNTHVPPTFGQGTKLEIKREDLRNVTPPKVSLFEPSKAEI
ANKQKATLVCLARGFFPDHVELSWWVNGKEVHSGVCTDPQAYKESNYSYCLSSRLRVSATFWHN
PRNHFRCQVQFHGLSEEDKWPEGSPKPVTQNISAEAWGRADCGITSASYQQGVLSATILYEILLGKA
TLYAVLVSTLVVMAMGGGGSGGGGSGGGGSRRDQRLPPDAHKPPGGGSFRTPIQEEQADAHSTLA
KI

SEQ ID NO:17          GPC3 STAR-CXCR2(TRAC-Nrec-Cys-TM) α chain
QVQLVQSGAEVKKPGASVKVSCKASGYTFTDYEMHWVRQAPGQGLEWMGALDPKTGDTAYSQ
KFKGRVTLTADESTSTAYMELSSLRSEDTAVYYCTRFYSYTYWGQGTLVTVSSDIQNPDPAVYQLR
DDSTLCLFTDFDSQINVPKTMESGTFITDKCVLDMKAMDSKSNGAIAWSNQTSFTCQDIFKETNAT
YPSSDVPCDATLTEKSFETDMNLNFQNLLVIVLRILLLKVAGFNLLMTLRLWSS

SEQ ID NO:18          GPC3 STAR-CXCR2(TRBC- Nrec-Cys-TM) β chain
DVVMTQSPLSLPVTPGEPASISCRSSQSLVHSNRNTYLHWYLQKPGQSPQLLIYKVSNRFSGVPDRF
SGSGSGTDFTLKISRVEAEDVGVYYCSQNTHVPPTFGQGTKLEIKRDLKNVFPPEVAVFEPSAEIATL
VCLARGFFPDHVELSWWVNGKEVHSGVCTDPQAYKESNYSYCLSSRLRVSATFWHNPRNHFRCQ
VQFHGLSEEDKWPEGSPKPVTQNISAEAWGRADCGITSASYQQGVLSATILYEILLGKATLYAVLVS
TLVVMAMVKRKNS

SEQ ID NO:19          GPC3 STAR-OX40-CXCR2 (TRAC- Nrec-Cys-TM) α chain
QVQLVQSGAEVKKPGASVKVSCKASGYTFTDYEMHWVRQAPGQGLEWMGALDPKTGDTAYSQ
KFKGRVTLTADESTSTAYMELSSLRSEDTAVYYCTRFYSYTYWGQGTLVTVSSDIQNPDPAVYQLR
DDSTLCLFTDFDSQINVPKTMESGTFITDKCVLDMKAMDSKSNGAIAWSNQTSFTCQDIFKETNAT
YPSSDVPCDATLTEKSFETDMNLNFQNLLVIVLRILLLKVAGFNLLMTLRLWGGGGSGGGGSGGGG
SRRDQRLPPDAHKPPGGGSFRTPIQEEQADAHSTLAKI

SEQ ID NO:20          GPC3 STAR-OX40-CXCR2(TRBC- Nrec-Cys-TM) β chain
DVVMTQSPLSLPVTPGEPASISCRSSQSLVHSNRNTYLHWYLQKPGQSPQLLIYKVSNRFSGVPDRF
SGSGSGTDFTLKISRVEAEDVGVYYCSQNTHVPPTFGQGTKLEIKRDLKNVFPPEVAVFEPSAEIATL
VCLARGFFPDHVELSWWVNGKEVHSGVCTDPQAYKESNYSYCLSSRLRVSATFWHNPRNHFRCQ
VQFHGLSEEDKWPEGSPKPVTQNISAEAWGRADCGITSASYQQGVLSATILYEILLGKATLYAVLVS
TLVVMAMGGGGSGGGGSGGGGSRRDQRLPPDAHKPPGGGSFRTPIQEEQADAHSTLAKI

SEQ ID NO:21          GPC3 CAR-CXCR2
DVVMTQSPLSLPVTPGEPASISCRSSQSLVHSNRNTYLHWYLQKPGQSPQLLIYKVSNRFSGVPDRF
SGSGSGTDFTLKISRVEAEDVGVYYCSQNTHVPPTFGQGTKLEIKRGGGGSGGGGSGGGGSQVQL
VQSGAEVKKPGASVKVSCKASGYTFTDYEMHWVRQAPGQGLEWMGALDPKTGDTAYSQKFKGR
VTLTADESTSTAYMELSSLRSEDTAVYYCTRFYSYTYWGQGTLVTVSSSRSHFVPVFLPAKPTTTPA
PRPPTPAPTIASQPLSLRPEACRPAAGGAVHTRGLDFACDIYIWAPLAGTCGVLLLSLVITKRGRKKL
LYIFKQPFMRPVQTTQEEDGCSCRFPEEEEGGCELRVKFSRSADAPAYQQGQNQLYNELNLGRREE
YDVLDKRRGRDPEMGGKPRRKNPQEGLYNELQKDKMAEAYSEIGMKGERRRGKGHDGLYQGLS
TATKDTYDALHMQALPPR

SEQ ID NO:22          heavy chain variable region CDR1 amino acid sequence
GYTFTDYE

SEQ ID NO:23          heavy chain variable region CDR2 amino acid sequence
LDPKTGDT

SEQ ID NO:24          heavy chain variable region CDR3 amino acid sequence
TR

SEQ ID NO:25          light chain variable region CDR1 amino acid sequence
QSLVHSNRNTY

SEQ ID NO:26          light chain variable region CDR2 amino acid sequence
KVS

SEQ ID NO:27 light chain variable region CDR3 amino acid sequence
SQNTHVP

SEQ ID NO:28 Endodomain-deleted mouse T cell receptor α chain constant region
DIQNPEPAVYQLKDPRSQDSTLCLFTDFDSQINVPKTMESGTFITDKTVLDMKAMDSKSNGAIAWS
NQTSFTCQDIFKETNATYPSSDVPCDATLTEKSFETDMNLNFQNLSVMGLRILLLKVAGFNLLMTLR
LW

SEQ ID NO:29 Endodomain-deleted mouse T cell receptor α chain constant region with cysteine
substitution and hydrophobic modification
DIQNPEPAVYQLKDPRSQDSTLCLFTDFDSQINVPKTMESGTFITDKCVLDMKAMDSKSNGAIAWS
NQTSFTCQDIFKETNATYPSSDVPCDATLTEKSFETDMNLNFQNLLVIVLRILLLKVAGFNLLMTLRL
W

SEQ ID NO:30 Endodomain-deleted mouse T cell receptor α chain constant region with N-terminal
modification, cysteine substitution and modification of transmembrane hydrophobic region
DIQNPDPAVYQLRDDSTLCLFTDFDSQINVPKTMESGTFITDKCVLDMKAMDSKSNGAIAWSNQTS
FTCQDIFKETNATYPSSDVPCDATLTEKSFETDMNLNFQNLLVIVLRILLLKVAGFNLLMTLRLW

SEQ ID NO:31 Endodomain-deleted mouse T cell receptor β chain constant region
DLRNVTPPKVSLFEPSKAEIANKQKATLVCLARGFFPDHVELSWWVNGKEVHSGVSTDPQAYKES
NYSYCLSSRLRVSATFWHNPRNHFRCQVQFHGLSEEDKWPEGSPKPVTQNISAEAWGRADCGITS
ASYQQGVLSATILYEILLGKATLYAVLVSTLVVMAM

SEQ ID NO:32 Endodomain-deleted mouse T cell receptor β chain constant region with cysteine
substitution
DLRNVTPPKVSLFEPSKAEIANKQKATLVCLARGFFPDHVELSWWVNGKEVHSGVCTDPQAYKES
NYSYCLSSRLRVSATFWHNPRNHFRCQVQFHGLSEEDKWPEGSPKPVTQNISAEAWGRADCGITS
ASYQQGVLSATILYEILLGKATLYAVLVSTLVVMAM

SEQ ID NO:33 Endodomain-deleted mouse T cell receptor β chain constant region with N-terminal
modification and cysteine substitution
DLKNVFPPEVAVFEPSAEIATLVCLARGFFPDHVELSWWVNGKEVHSGVCTDPQAYKESNYSYCLS
SRLRVSATFWHNPRNHFRCQVQFHGLSEEDKWPEGSPKPVTQNISAEAWGRADCGITSASYQQGV
LSATILYEILLGKATLYAVLVSTLVVMAM

SEQ ID NO:34 (G4S)3 linker
GGGGSGGGGSGGGGS

## Claims

1. An isolated therapeutic T cell co-expressing CXCR2 and a Synthetic T cell Receptor and Antigen Receptor (STAR) against GPC3, wherein the STAR against GPC3 comprises an antigen binding region against GPC3, and the STAR against GPC3 comprises an α chain comprising a first constant region and a β chain comprising a second constant region.

2. The isolated therapeutic T cell according to claim 1, wherein
i) the α chain comprises an antigen binding region against GPC3 and a first constant region, the β chain comprises a second constant region; or
ii) the α chain comprises a first constant region, the β chain comprises an antigen binding region against GPC3 and a second constant region;
iii) the α chain comprises a first antigen binding region against GPC3 and a first constant region, the β chain comprises a second antigen binding region against GPC3 and a second constant region.

3. The isolated therapeutic T cell according to claim 1 or 2, wherein the first constant region is a native TCR α chain constant region, e.g., a native human TCR α chain constant region or a native mouse TCR α chain constant region.

4. The isolated therapeutic T cell according to claim 1 or 2, wherein the first constant region is a modified TCR α chain constant region.

5. The isolated therapeutic T cell according to claim 4, wherein the modified TCRα chain constant region is derived from the mouse TCRα chain constant region, and the amino acid at position 48, such as threonine T, is mutated to cysteine C, as compared to the wild-type mouse TCRα chain constant region.

6. The isolated therapeutic T cell according to claim 4 or 5, wherein the modified TCR α chain constant region is derived from the mouse TCR α chain constant region, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCRα chain constant region.

7. The isolated therapeutic T cell according to any one of claims 4-6, wherein the modified TCR α chain constant region is derived from the mouse TCR α chain constant region, the amino acid at position 6 such as E is substituted by D, and the amino acid K at position 13 is substituted by R, and amino acids at positions 15-18 are deleted, as compared to the wild-type mouse TCRα chain constant region.

8. The isolated therapeutic T cell according to any one of claims 4-7, wherein the modified TCRα chain constant region is derived from the mouse TCRα chain constant region, the amino acid at position 48, such as threonine T, is mutated to Cysteine C, the amino acid at position 112 such as serine S is mutated to leucine L, the amino acid at position 114 such as methionine M is mutated to isoleucine I, the amino acid at position 115 such as Glycine G is mutated to valine V, as compared to the wild-type mouse TCRα chain constant region.

9. The isolated therapeutic T cell according to any one of claims 4-8, wherein the modified TCRα chain constant region is derived from the mouse TCRα chain constant region, the amino acid at position 6, such as E, is substituted by D, and the amino acid K at position 13 is substituted by R, the amino acids at positions 15-18 are deleted, the amino acid at position 48 such as threonine T is mutated to cysteine C, and the amino acid at position 112 such as serine S is mutated to leucine acid L, the amino acid at position 114 such as methionine M is mutated to isoleucine I, and the amino acid at position 115 such as glycine G is mutated to valine V, as compared to the wild-type mouse TCRα chain constant region.

10. The isolated therapeutic T cell according to any one of claims 4-9, wherein the modified TCR α chain constant region is derived from the mouse TCR α chain constant region, and lacks the endodomain of the constant region, for example, the amino acids at positions 136-137 are deleted, as compared to the wild-type mouse TCRα chain constant region.

11. The isolated therapeutic T cell according to claim 4, wherein the modified TCRα chain constant region comprises an amino acid sequence shown in one of SEQ ID NOs: 3-4 and 28-30.

12. The isolated therapeutic T cell according to any one of claims 1-11, wherein the second constant region is a native TCR β chain constant region, e.g., a native human TCR β chain constant region or a native mouse TCR β chain constant region.

13. The isolated therapeutic T cell according to any one of claims 1-11, wherein the second constant region is a modified TCR β chain constant region.

14. The isolated therapeutic T cell according to claim 13, wherein the modified TCR β chain constant region is derived from the mouse TCR β chain constant region, and the amino acid at position 56, such as serine S, is mutated to cysteine C, as compared to the wild-type mouse TCRβ chain constant region.

15. The isolated therapeutic T cell according to any one of claims 13-14, wherein the modified TCR β chain constant region is derived from the mouse TCR β chain constant region, the amino acid at position 3 such as R is substituted by K, the amino acid at position 6 such as T is substituted by F, the amino acid K at position 9 is substituted by E, S at position 11 is substituted by A, L at position 12 is substituted by V, and amino acids at positions 17 and 21-25 are deleted, as compared to the wild-type mouse TCRβ chain constant region.

16. The isolated therapeutic T cell according to any one of claims 13-15, wherein the modified TCR β chain constant region is derived from a mouse TCR β chain constant region, the amino acid at position 56 such as serine S is mutated to cysteine C, the amino acid at position 3 such as R is substituted by K, the amino acid at position 6 such as T is substituted by F, K at position 9 is substituted by E, S at position 11 is substituted by A, and L at position 12 is substituted by V, and amino acids 17, 21-25 are deleted, as compared to the wild-type mouse TCRβ chain constant region.

17. The isolated therapeutic T cell according to any one of claims 13-16, wherein the modified TCR β chain constant region is derived from the mouse TCR beta chain constant region, and lacks the endodomain of the constant region, for example, the amino acids at positions 167-172 are deleted, as compared to the wild-type mouse TCRβ chain constant region.

18. The isolated therapeutic T cell according to claim 13, wherein the modified TCRβ chain constant region comprises an amino acid sequence shown in one of SEQ ID NOs:7-8 and 31-33.

19. The isolated therapeutic T cell according to any one of claims 1-18, wherein
i) the antigen-binding region against GPC3 comprises a heavy chain variable region of an antibody specifically binding to the target antigen GPC3 and a light chain variable region of said antibody, for example, the heavy chain variable region and the light chain variable region are linked by a linker;
ii) the first antigen binding region comprises a heavy chain variable region of an antibody that specifically binds to target antigen GPC3, and the second antigen binding region comprises a light chain variable region of said antibody; or
iii) the first antigen binding region comprises a light chain variable region of an antibody that specifically binds to target antigen GPC3, and the second antigen binding region comprises a heavy chain variable region of said antibody.

20. The isolated therapeutic T cell according to claim 19, wherein the heavy chain variable region comprises VH CDR1 shown in SEQ ID NO:22, VH CDR2 shown in SEQ ID NO:23, and VH CDR3 shown in SEQ ID NO:24, the light chain variable region comprises VL CDR1 shown in SEQ ID NO:25, VL CDR2 shown in SEQ ID NO:26, and VL CDR3 shown in SEQ ID NO:27, or
wherein the heavy chain variable region comprises the amino acid sequence shown in SEQ ID NO:9, and the light chain variable region comprises the amino acid sequence shown in SEQ ID NO:10.

21. The isolated therapeutic T cell according to any one of claims 1-20, wherein the α chain and/or β chain has at least one exogenous intracellular functional domain linked to its C-terminus.

22. The isolated therapeutic T cell according to claim 21, wherein the exogenous intracellular functional domain is linked directly or via a linker to the C-terminus of the constant region of the α chain and/or β chain,
preferably, the exogenous intracellular functional domain is linked to the C-terminus of the constant region of the α-chain and/or β-chain whose endodomain is deleted, through a linker,
preferably, the linker is a (G4S)n linker, where n represents an integer from 1-10, preferably, n is 3.

23. The isolated therapeutic T cell according to claim 22, wherein the exogenous intracellular functional domain is an endodomain of a co-stimulatory molecule, preferably an endodomain of OX40, for example, the endodomain of OX40 comprises the amino acid sequence of SEQ ID NO:11.

24. The isolated therapeutic T cell according to any one of claims 1-23, wherein the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; and optionally, the α chain comprises an endodomain of OX40 linked to the C-terminus of the constant region.

25. The isolated therapeutic T cell according to any one of claims 1-23, wherein the α chain comprises a first antigen binding region against GPC3 and a first constant region, wherein the first antigen binding region against GPC3 comprises the amino acid sequence of the heavy chain variable region shown in SEQ ID NO:9; the first constant region is a modified TCRα chain constant region, which is derived from a mouse TCRα chain constant region, and the amino acid at position 6 such as E is substituted by D, K at position 13 is substituted by R, the amino acids at positions 15-18 are deleted, the amino acid at position 48 for example, Threonine T is mutated to cysteine C, the amino acid at position 112 such as serine S is mutated to leucine L, the amino acid at position 114 such as methionine M is mutated to isoleucine I, the amino acid at position 115, such as glycine G, is mutated to valine V, as compared to the wild-type mouse TCRα chain constant region; optionally, the α chain comprises an endodomain of OX40 linked to the C-terminus of the constant region.

26. The isolated therapeutic T cell according to any one of claims 24-25, wherein the modified TCR α chain constant region lacks the endodomain of the constant region, for example, amino acids at positions 136-137 are deleted, as compared to the wild-type mouse TCRα chain constant region.

27. The isolated therapeutic T cell according to any one of claims 1-26, wherein the α chain comprises an amino acid sequence shown in SEQ ID NO: 13, 15, 17 or 19.

28. The isolated therapeutic T cell according to any one of claims 1-27, wherein the β chain comprises a second antigen binding region against GPC3 and a second constant region, wherein the second antigen binding region against GPC3 comprises the amino acid sequence of the light chain variable region shown in SEQ ID NO:10; the second constant region is a modified TCRβ chain constant region, which is derived from a mouse TCRβ chain constant region, and the amino acid at position 56 such as serine S is mutated to cysteine C, as compared to the wild type mouse TCRβ chain constant region; and optionally, the β chain comprises an endodomain of OX40 linked to the C-terminus of the constant region.

29. The isolated therapeutic T cell according to any one of claims 1-27, wherein the β chain comprises a second antigen binding region against GPC3 and a second constant region, wherein the second antigen binding region against GPC3 comprises the amino acid sequence of the light chain variable region shown in SEQ ID NO:10; the second constant region is a modified TCRβ chain constant region, which is derived from a mouse TCRβ chain constant region, and the amino acid at position 56 such as serine S is mutated to cysteine C, the amino acid at position 3, such as R, is substituted by K, the amino acid at position 6, such as T, is substituted by F, K at position 9 is substituted by E, S at position 11 is substituted by A, L at position 12 is substituted by V, and amino acids at positions 17 and 21-25 are deleted, as compared to the wild type mouse TCRβ chain constant region; and optionally, the β chain comprises an endodomain of OX40 linked to the C-terminus of the constant region.

30. The isolated therapeutic T cell according to any one of claims 28-29, wherein the modified TCR β chain constant region lacks the endodomain of the constant region, for example, amino acids at positions 167-172 are deleted, as compared to the wild-type mouse TCRβ chain constant region.

31. The isolated therapeutic T cell according to any one of claims 1-30, wherein the β chain comprises an amino acid sequence shown in SEQ ID NO: 14, 16, 18 or 20.

32. The isolated therapeutic T cell according to claim 1, wherein the STAR comprises an α chain set forth in SEQ ID NO:13 and a β chain set forth in SEQ ID NO:14; or the STAR comprises an α chain set forth in SEQ ID NO:15 and a β chain set forth in SEQ ID NO:16; or the STAR comprises an α chain set forth in SEQ ID NO:17 and a β chain set forth in SEQ ID NO:18; or the STAR comprises an α chain set forth in SEQ ID NO:19 and a β chain set forth in SEQ ID NO:20; or the STAR comprises an α chain set forth in SEQ ID NO:15 and a β chain set forth in SEQ ID NO:14; or the STAR comprises an α chain set forth in SEQ ID NO:19 and a β chain set forth in SEQ ID NO:18.

33. A pharmaceutical composition, which comprises the therapeutic T cell of any one of claims 1-32 and a pharmaceutically acceptable carrier.

34. Use of the therapeutic T cell of any one of claims 1-32 or the pharmaceutical composition of claim 33 in the preparation of a medicine for the treatment of a disease such as cancer in a subject.

35. The use of claim 34, wherein the disease is a GPC3-related disease, such as liver cancer such as hepatocellular carcinoma, lung cancer such as lung squamous cell carcinoma (SqCC), gastric cancer, ovarian cancer, melanoma or pediatric embryonal tumor.
